# EUROPEAN PATENT APPLICATION

(11) **EP 4 768 504 A1**
(43) Date of publication of application: **01.07.2026**
(21) Application number: 24856850.3
(22) Date of filing: 23.08.2024
(51) Int. Cl.: C07K 16/24, A61P 29/00, A61P 19/02, A61P 1/00, A61P 17/06, A61K 39/00

(54) **METHOD FOR TREATING TNF-ALPHA-RELATED DISEASES**

(30) Priority: 23.08.2023 KR 20230110838
(71) Applicant: Celltrion, Inc., Incheon 22014 (KR)
(72) Inventor: LEE, Juhyun, Incheon 22014 (KR); LEE, Sunhee, Incheon 22014 (KR); LEE, Jimin, Incheon 22014 (KR)
(74) Representative: Maiwald GmbH
(86) International application number: PCT/KR2024/012605
(87) International publication number: WO 2025/042239

(57) **Abstract**

The present invention relates to a method for treating TNFα-related diseases by subcutaneously administering an antibody (anti-TNFα antibody) that binds to TNFα or an antigen-binding fragment thereof. A treatment method, a composition, a kit, or a use according to the present invention provides the advantage that administration time is reduced compared to intravenous injections and the amount of time that patients stay in hospitals is reduced, and thus patient satisfaction is increased through improved convenience and improved quality of life. In particular, the present invention has the advantage of providing a therapeutic agent that is highly effective in improving medication convenience, disease treatment effects, growth disorders, and the like with respect to pediatric/adolescent patients suffering from autoimmune diseases.

## Description

### Technical Field

The present application relates to a method for treating TNFα-related diseases by subcutaneously administering an antibody that binds to TNFα (anti-TNFα antibody).

### Background Art

Tumor necrosis factor alpha (TNFα) is a cell signaling protein (cytokine) involved in systemic inflammation, and is one of the cytokines that form an acute phase response. TNFα is associated with various diseases and disorders including sepsis, infection, autoimmune disease, and transplant rejection. TNFα promotes an immune response, which causes many clinical problems associated with an autoimmune abnormality such as rheumatoid arthritis, ankylosing spondylitis, ulcerative colitis, adult Crohn's disease, pediatric Crohn's disease, psoriasis, and psoriatic arthritis.

Meanwhile, inflammatory bowel disease (IBD), such as ulcerative colitis and Crohn's disease, among autoimmune diseases, is known to occur in young adults, and in particular, approximately 25% of IBD patients develop the disease at an age of less than 20 years. The prevalence of IBD is increasing worldwide, and the prevalence of pediatric-adolescent IBD in the United States is known to be 100 to 200 per 100,000 pediatric-adolescent population. Moreover, 10 to 40% of pediatric-adolescent IBD patients have a growth disorder, and IBD is characterized in that the lower the age of onset of IBD, the higher the probability of undergoing surgery due to complications and the likelihood of disease recurrence. Autoimmune disease such as inflammatory bowel disease can be treated using a TNFα inhibitor, and early treatment of autoimmune disease for pediatric-adolescent patients is known to improve treatment outcomes for the patient, reduce reductions in height and weight gainof the patient, and improve a developmental delay of skeleton, etc. However, currently, the only TNFα inhibitor approved in the United States as a therapeutic agent for pediatric-adolescent IBD is infliximab, administered by intravenous injection and adalimumab administered by subcutaneous injection.

Infliximab is a kind of chimeric monoclonal antibodies capable of serving as a TNFα inhibitor, and Remsima, Remicade, Renflexis and the like are commercially available, but all of these products are all produced as lyophilized powder, and are administered to patients by intravenous injection according to an administration usage and dose for each disease after being redissolved and diluted. However, the intravenous administration causes a considerable burden and inconvenience on patients' daily lives because patients must visit a hospital for medication, and it takes 2 to 4 hours, including waiting time. Furthermore, it is problematic in that an administrator is limited to a person who has received medical training. Therefore, subcutaneous (SC) administration is proposed as an alternative administration route, and the subcutaneous administration allows trained patients to administer the drug by themselves, and can shorten administration time from 30 to 90 minutes to 2 to 5 minutes. Nevertheless, currently, infliximab for subcutaneous administration has not yet been commercially available as a therapeutic agent for a pediatric-adolescent autoimmune disease (e.g., IBD) in various countries such as the United States.

Meanwhile, an antibody drug can be a product suitable for subcutaneous administration only if the drug should be a stable liquid formulation containing a high concentration of the antibody, and even for such a product, the efficacy and safety of the formulation should be proven through clinical trials.

However, it is known that as the molecular weight of a substance to be subcutaneously administered increases, the bioavailability thereof in subcutaneous administration decreases. Thus, it is very difficult to develop a product for subcutaneous administration and determine a suitable administration dose/administration cycle thereof. Furthermore, when subcutaneously administering an antibody protein, which is a high-molecular-weight substance, a problem occurs in that bioavailability is lowered due to various factors such as blood vessels and lymphatic vessels within skin tissue, proteolytic enzymes, and the like. Moreover, there are problems in that as the stratum corneum of a skin epithelium is thicker, the absorption of the subcutaneously administered substance is more hindered, and as the body surface area relative to body weight is greater, the absorption of the subcutaneously administered substance is more excessively promoted.

Therefore, the administration dose and/or administration cycle of an antibody protein for subcutaneous administration for treating a patient in a pediatric and/or adolescent period, who is known to have a large body surface area relative to body weight, cannot be same as the subcutaneous administration dose and/or administration cycle of an antibody protein for adults. To determine the administration dose/administration cycle, it is necessary to determine carefully by considering various factors.

The present applicant has completed a subcutaneous administration therapy which exhibits efficacy and stability equivalent to a formulation for intravenous administration in subcutaneous administration of an anti-TNFα antibody, for example, an infliximab formulation, thereby improving administration convenience of a patient, for example, a patient in a pediatric and/or adolescent period (that is, a pediatric-adolescent patient) and improving the quality of life.

### Disclosure of Invention

### Technical Problem

An objective of the present invention is to provide a method for treating a TNFα-related disease by subcutaneously administering to a subject, particularly a subject who is 20 years old or less, preferably a pediatric-adolescent subject, a pharmaceutical composition containing an anti-TNFα antibody or an antigen-binding fragment thereof.

Another objective of the present invention is to provide a pharmaceutical composition for treating a TNFα-related disease, containing an anti-TNFα antibody or an antigen-binding fragment thereof, wherein the composition is subcutaneously administered to a subject, particularly a subject who is 20 years old or less, preferably a pediatric-adolescent subject.

Another objective of the present invention is to provide a kit including: a pharmaceutical composition containing an anti-TNFα antibody or an antigen-binding fragment thereof; and an instruction indicating that the pharmaceutical composition is subcutaneously administered to a subject, particularly a subject who is 20 years old or less, preferably a pediatric-adolescent subject, for treating a TNFα-related disease.

Another objective of the present invention is to provide a use of an anti-TNFα antibody or an antigen-binding fragment thereof in manufacturing a medicament for treating a TNFα-related disease, wherein the medicament is subcutaneously administered to a subject, particularly a subject who is 20 years old or less, preferably a pediatric-adolescent subject.

### Solution to Problem

The present invention provides a method for treating a TNFα-related disease, the method including subcutaneously administering to a subject, particularly a subject who is 20 years old or less, preferably a pediatric-adolescent subject, a pharmaceutical composition containing an anti-TNFα antibody or an antigen-binding fragment thereof.

In addition, the present invention provides a pharmaceutical composition for treating a TNFα-related disease, the pharmaceutical composition containing an anti-TNFα antibody or an antigen-binding fragment thereof, wherein the composition is subcutaneously administered to a subject, particularly a subject who is 20 years old or less, preferably a pediatric-adolescent subject.

In addition, the present invention provides a kit including: (a) a pharmaceutical composition containing an anti-TNFα antibody or an antigen-binding fragment thereof and a pharmaceutically acceptable carrier; and (b) an instruction indicating that the pharmaceutical composition is subcutaneously administered to a subject, particularly a subject who is 20 years old or less, preferably a pediatric-adolescent subject, for treating a TNFα-related disease.

In addition, the present invention provides a use of an anti-TNFα antibody or an antigen-binding fragment thereof in manufacturing a medicament for treating a TNFα-related disease by being subcutaneously administered to a subject, particularly a subject who is 20 years old or less, preferably a pediatric-adolescent subject.

In one embodiment of the present invention, the anti-TNFα antibody may include one or more selected from the group consisting of infliximab, adalimumab, certolizumab pegol, golimumab, and biosimilars thereof.

In one embodiment of the present invention, the anti-TNFα antibody may be infliximab.

In one embodiment of the present invention, the anti-TNFα antibody may include a chimeric human-mouse IgG monoclonal antibody.

In one embodiment of the present invention, the anti-TNFα antibody may include: a light chain variable region including a CDR1 domain including an amino acid sequence of SEQ ID NO: 1, a CDR2 domain including an amino acid sequence represented by YAS, and a CDR3 domain including an amino acid sequence of SEQ ID NO: 2; and a heavy chain variable region including a CDR1 domain including an amino acid sequence of SEQ ID NO: 3, a CDR2 domain including the amino acid sequence of SEQ ID NO: 4, and a CDR3 domain including an amino acid sequence of SEQ ID NO: 5.

In one embodiment of the present invention, the anti-TNFα antibody may include a light chain variable region including an amino acid sequence of SEQ ID NO: 6, and a heavy chain variable region including an amino acid sequence of SEQ ID NO: 7.

In one embodiment of the present invention, the anti-TNFα antibody may include a light chain including an amino acid sequence of SEQ ID NO: 8, and a heavy chain including an amino acid sequence of SEQ ID NO: 9.

In one embodiment of the present invention, the composition may comprise: a surfactant; a sugar or derivative thereof; and a buffer containing acetate or histidine.

In one embodiment of the present invention, the composition may comprise, as a surfactant, polysorbate 20, polysorbate 40, polysorbate 60, polysorbate 80, or a mixture thereof.

In one embodiment of the present invention, a concentration of the surfactant in the composition may be 0.02 to 0.1 % (w/v).

In one embodiment of the present invention, the composition may comprise sorbitol, mannitol, trehalose, sucrose, or a mixture thereof as the sugar or derivative thereof.

In one embodiment of the present invention, a concentration of the sugar or derivative thereof in the composition may be 1 to 10 % (w/v).

In one embodiment of the present invention, the composition may comprise acetate as a buffer.

In one embodiment of the present invention, a concentration of the buffer in the composition may be 1 to 50 mM.

In one embodiment of the present invention, the composition may have a pH of 4.0 to 5.5.

In one embodiment of the present invention, the composition may comprise: (A) 90 to 220 mg/ml or 90 to 180 mg/ml of an anti-TNFα antibody; (B) 0.02 to 0.1 % (w/v) of polysorbate; (C) 1 to 10 % (w/v) of sorbitol; and (D) 1 to 90 mM or 1 to 50 mM of a buffer containing acetate or histidine.

In one embodiment of the present invention, the composition may not comprise aspartic acid, lysine, arginine, or a mixture thereof.

In one embodiment of the present invention, the composition may not comprise NaCl, KCl, NaF, KBr, NaBr, Na₂SO₄, NaSCN, K₂SO₄, or a mixture thereof.

In one embodiment of the present invention, the composition may not comprise a chelating agent.

In one embodiment of the present invention, the composition may have a viscosity of 0.5 cP to 10.0 cP after 1 month at a temperature of 40°C ± 2°C, or a viscosity of 0.5 cP to 5 cP after 6 months at a temperature of 5°C ± 3°C.

In one embodiment of the present invention, the composition may not undergo, prior to use, a reconstitution step, a dilution step, or both thereof.

In one embodiment of the present invention, the composition may be filled in a pre-filled syringe or auto-injector and administered to a subject.

In one embodiment of the present invention, the subject may include a mammal.

In one embodiment of the present invention, the subject may include a human.

In one embodiment of the present invention, the TNFα-related disease may include rheumatoid arthritis, ulcerative colitis, Crohn's disease, plaque psoriasis, psoriatic arthritis, and ankylosing spondylitis.

In one embodiment of the present invention, the antibody or antigen-binding fragment thereof may be subcutaneously administered at a dose of 40 to 300 mg.

In one embodiment of the present invention, the antibody or antigen-binding fragment thereof may be subcutaneously administered at a dose of 40 to 180 mg.

In one embodiment of the present invention, the antibody or antigen-binding fragment thereof may be subcutaneously administered at a dose of 40 mg, 60 mg, 90 mg, 120 mg, 180 mg, or 240 mg.

In one embodiment of the present invention, the antibody or antigen-binding fragment thereof may be subcutaneously administered at a dose of 40 to 120 mg.

In one embodiment of the present invention, the antibody or antigen-binding fragment thereof may be subcutaneously administered at a dose of 40, 50, 60, 70, 80, 90, 100, 110, 120, 130, 140, 150, 160, 170, 180, 190, 200, 210, 220, 230, 240, 250, 260, 270, 280, 290, or 300 mg.

In one embodiment of the present invention, the subject may be a person who is 20 years old or less, 19 years old or less, 18 years old or less, 17 years old or less, 2 to 18 years old, 2 to 17 years old, or 6 to 17 years old, or a pediatric-adolescent subject. In one embodiment of the present invention, when a subject's condition is not improved or a therapeutic response is lost, a dose of the anti-TNFα antibody or antigen-binding fragment thereof may be increased and subcutaneously administered.

In one embodiment of the present invention, when the TNFα-related disease is rheumatoid arthritis, the anti-TNFα antibody or antigen-binding fragment thereof may be subcutaneously administered to the subject at a dose of 40 to 180 mg.

In one embodiment of the present invention, when the TNFα-related disease is any one or more selected from the group consisting of ulcerative colitis, Crohn's disease, plaque psoriasis, psoriatic arthritis, and ankylosing spondylitis, the anti-TNFα antibody or antigen-binding fragment thereof may be subcutaneously administered to the subject at a dose of 40 to 240 mg. In one embodiment of the present invention, when the subject having the TNFα-related disease is a subject who is 20 years old or less, 19 years old or less, 18 years old or less, 17 years old or less, 2 to 18 years old, 2 to 17 years old, or 6 to 17 years old, or a pediatric-adolescent subject, the anti-TNFα antibody or antigen-binding fragment thereof may be subcutaneously administered to the subject at a dose of 40 to 120 mg. Preferably, the TNFα-related disease may be one or more diseases selected from the group consisting of ulcerative colitis and Crohn's disease.

In one embodiment of the present invention, when a body weight of the subject is less than 80 kg, the antibody or antigen-binding fragment thereof may be subcutaneously administered at a dose of 90 to 180 mg; and when a body weight of the subject is 80 kg or more, the antibody or antigen-binding fragment thereof may be subcutaneously administered at a dose of 190 to 270 mg.

In one embodiment of the present invention, when a body weight of the subject is 40 kg or more and/or the subject is 20 years old or less, 19 years old or less, 18 years old or less, 17 years old or less, 2 to 18 years old, 2 to 17 years old, or 6 to 17 years old or a pediatric-adolescent subject, the anti-TNFα antibody or antigen-binding fragment thereof may be subcutaneously administered at a dose of 60 to 120 mg; and when a body weight of the subject is 40 kg or less, the anti-TNFα antibody or antigen-binding fragment thereof may be subcutaneously administered at a dose of 40 to 60 mg. Preferably, the TNFα-related disease may be one or more diseases selected from the group consisting of ulcerative colitis and Crohn's disease.

In one embodiment of the present invention, when a body weight of the subject is 40 kg or more, 120 mg of the anti-TNFα antibody or antigen-binding fragment thereof may be subcutaneously administered five times, and then, 60 mg of the anti-TNFα antibody or an antigen-binding fragment thereof may be subcutaneously administered from a 6-th administration. Preferably, the TNFα-related disease may be one or more diseases selected from the group consisting of ulcerative colitis and Crohn's disease.

In one embodiment of the present invention, when a body weight of the subject is 40 kg or less, 60 mg of the anti-TNFα antibody or antigen-binding fragment thereof may be subcutaneously administered five times, and then, 40 mg of the anti-TNFα antibody or antigen-binding fragment thereof may be subcutaneously administered from a 6-th administration. Preferably, the TNFα-related disease may be one or more diseases selected from the group consisting of ulcerative colitis and Crohn's disease. In one embodiment of the present invention, the antibody or antigen-binding fragment thereof may be administered at intervals of 1 to 8 weeks.

In one embodiment of the present invention, the antibody or antigen-binding fragment thereof may be subcutaneously administered at intervals of 1, 2, 3, 4, 5, 6, 7, or 8 weeks.

In one embodiment of the present invention, the antibody or antigen-binding fragment thereof may be subcutaneously administered at intervals of 2 or 4 weeks.

In one embodiment of the present invention, the antibody or an antigen-binding fragment thereof may be subcutaneously administered at intervals of 2 weeks.

In one embodiment of the present invention, the subject to be administered with the anti-TNFα antibody may include one or more of the following characteristics:
a) a subject who has an insufficient response to a disease-modifying anti-rheumatic drug (DMARD) including methotrexate;
b) a subject who has not been treated with methotrexate or another DMARD previously;
c) a subject who exhibits an increase in a serological indicator related to severe axial symptoms and inflammation, and who does not exhibit an adequate response to a conventional treatment;
d) a subject who does not exhibit an adequate response to a systemic therapy including methotrexate, cyclosporine, or psoralen ultraviolet A therapy (PUVA), and does not have tolerance to such a therapy, or for whom such a therapy is contraindicated;
e) a subject who does not exhibit an adequate response to a treatment of corticosteroid, 6-mercaptopurine, azathioprine, or an immunosuppressant, and does not have tolerance to such a treatment, or for whom such a treatment method is contraindicated; or
f) a subject who does not exhibit a response to a conventional treatment including an antibiotic, excretion, or an immunosuppressive treatment.

In one embodiment of the present invention, the subject may be a subject who has been intravenously administered with the anti-TNFα antibody or antigen-binding fragment thereof at least once prior to subcutaneous administration.

In one embodiment of the present invention, the subject may be a subject who has been intravenously administered with the anti-TNFα antibody or antigen-binding fragment thereof two or three times prior to subcutaneous administration.

In one embodiment of the present invention, a) in the case where a subject has a rheumatoid arthritis, the subject may be a subject who has been intravenously administered with the anti-TNFα antibody or antigen-binding fragment thereof two times prior to subcutaneous administration, and b) in the case where a subject has any one or more diseases selected from the group consisting of ulcerative colitis, Crohn's disease, plaque psoriasis, psoriatic arthritis, and ankylosing spondylitis, the subject may be a subject who has been intravenously administered with the anti-TNFα antibody or antigen-binding fragment thereof two or three times prior to subcutaneous administration.

In one embodiment of the present invention, the subject may be a subject who has been intravenously administered with the anti-TNFα antibody or antigen-binding fragment thereof two times at 0-week and 2-week, or intravenously administered three times at weeks 0, 2, and 6, prior to subcutaneous administration.

In one embodiment of the present invention, the subject may be a subject who has been intravenously administered with the anti-TNFα antibody or antigen-binding fragment thereof at a dose of 1 to 10 mg/kg per administration prior to subcutaneous administration.

In one embodiment of the present invention, the subject may be a subject who has been intravenously administered with the anti-TNFα antibody or antigen-binding fragment thereof at a dose of 3 to 5 mg/kg per administration prior to subcutaneous administration.

In one embodiment of the present invention, a) in the case where a subject has a rheumatoid arthritis, the subject may be a subject who has been intravenously administered with the anti-TNFα antibody or antigen-binding fragment thereof at a dose of 3 mg/kg per administration, and b) in the case where a subject has any one or more diseases selected from the group consisting of ulcerative colitis, Crohn's disease, plaque psoriasis, psoriatic arthritis, and ankylosing spondylitis, the subject may be a subject who has been intravenously administered with the anti-TNFα antibody or antigen-binding fragment thereof at a dose of 5 mg/kg per administration.

In one embodiment of the present invention, a first subcutaneous administration may be performed at 2 to 8 weeks after the last intravenous administration.

In one embodiment of the present invention, a first subcutaneous administration may be performed at 4 weeks after the last intravenous administration.

In one embodiment of the present invention, the composition containing the anti-TNFα antibody or antigen-binding fragment thereof may be administered together with, before or after administration of one or more selected from the group consisting of infliximab, adalimumab, certolizumab pegol, golimumab, and biosimilars thereof.

In one embodiment of the present invention, the anti-TNFα antibody or antigen-binding fragment thereof may be administered together with, before, or after administration of at least one selected from the group consisting of a disease-modifying antirheumatic drug (DMARD), a steroid, and an immunosuppressant. Specifically, the disease-modifying antirheumatic drug (DMARD) may be selected from the group consisting of methotrexate, leflunomide, sulfasalazine, and hydroxychloroquine, the steroid may be selected from the group consisting of corticosteroid, glucocorticoid, cortisol, mineralocorticoid, and aldosterone, and the immunosuppressant may be selected from the group consisting of azathioprine, 6-mercaptopurine, cyclosporin A, tacrolimus, mycophenolic acid, bredinin, an mTOR inhibitor, and an anti-lymphocyte antibody.

One embodiment of the present invention may be an administration method in which a trough blood concentration immediately before the next application (C_{trough}: minimum concentration immediately before the next application) of the anti-TNFα antibody or antigen-binding fragment thereof after subcutaneous administration to the subject is maintained at 0.01 µg/ml or more.

One embodiment of the present invention may be an administration method in which: a) in the case of a subject having a rheumatoid arthritis, a trough blood concentration (C_{trough}) of the anti-TNFα antibody or antigen-binding fragment thereof after subcutaneous administration is maintained at 1 µg/ml or more, and b) in the case of a subject having any one or more diseases selected from the group consisting of ulcerative colitis, Crohn's disease, plaque psoriasis, psoriatic arthritis, and ankylosing spondylitis, a trough blood concentration (C_{trough}) of the anti-TNFα antibody or antigen-binding fragment thereof after subcutaneous administration is maintained at 5 µg/ml or more.

In one embodiment of the present invention, after subcutaneous administration, the subject has any one or more of the following characteristics:
a) a disease activity score in 28 joints (DAS28) being reduced by at least 2.0; or
b) a Crohn's disease activity index (CDAI) being reduced by at least 70.

One embodiment of the present invention may provide a kit including: (a) a pharmaceutical composition containing an anti-TNFα antibody or an antigen-binding fragment thereof; and (b) an instruction indicating that the anti-TNFα antibody or antigen-binding fragment thereof is subcutaneously administered at a dose of 40 to 300 mg at intervals of 1 to 8 weeks in order to treat a subject with a TNFα-related disease.

One embodiment of the present invention may provide a use of an anti-TNFα antibody or an antigen-binding fragment thereof for manufacturing a medicament for treatment of a TNFα-related disease by being administered to a subject, wherein the anti-TNFα antibody or antigen-binding fragment thereof is subcutaneously administered at a dose of 40 to 300 mg at intervals of 1 to 8 weeks.

### Advantageous Effects of Invention

A treatment method, a composition, a kit, or a use according to the present invention is capable of treating TNFα-related diseases by subcutaneously administering an anti-TNFα antibody or an antigen-binding fragment thereof. Furthermore, the treatment method, composition, kit, or use according to the present invention provides advantages of improving convenience and quality of life by reducing administration time and the time required for patients to stay in hospital compared to intravenous injection, thereby increasing patient satisfaction. Moreover, the present invention provides an advantage of providing a therapeutic agent that has an excellent effect in improving medication convenience and disease treatment efficacy, and alleviating a growth disorder in a patient who has suffered from an autoimmune disease during a pediatric-adolescent period.

Furthermore, the treatment method, composition, kit, or use according to the present invention is added as a new treatment option involving an anti-TNFα antibody such as infliximab, thereby providing an advantage of not causing burden and resistance to drug changes to a patient who has previously received infliximab by intravenous injection and a healthcare provider.

### Brief Description of Drawings

Fig. 1 is a simulated graph showing a median (prediction interval 5th-95th percentile) of blood infliximab concentrations over time by body weight strata when infliximab is intravenously administered to patients with Crohn's disease and ulcerative colitis at a dose of 5 mg/kg at 0-week, 2-week, and 6-week, and then subcutaneously administered every 2 weeks starting at 10-week.
Fig. 2 shows the properties (Fig. 2a) and turbidity (Fig. 2b) of a formulation containing high concentration of infliximab after being stored at 5°C and 25°C for 0, 4, 8, or 12 weeks.
Fig. 3 shows results of measuring the antigen-binding ability of an antibody by using ELISA (Fig. 3a) and a result of measuring immunoglobulin content, heavy chain content, and light chain content by using CE-SDS (Figs. 3b and 3c) after storing a formulation containing high concentration of infliximab at 5°C and 25°C for 0, 4, 8, or 12 weeks.
Fig. 4 shows results of measuring peak values by using IEC-HPLC (Fig. 4a) and a result confirming the number of particles by using MFI (Fig. 4b) after storing a formulation containing high concentration of infliximab at 5°C and 25°C for 0, 4, 8, or 12 weeks.
Fig. 5 shows results of particle counting using a HIAC particle counter after storing a formulation containing high concentration of infliximab at 5°C and 25°C for 0, 4, 8, or 12 weeks.

### Best Mode for Carrying out the Invention

The present invention relates to a method for treating a TNFα-related disease, the method including subcutaneously administering to a subject, particularly a subject who is 20 years old or less, preferably a pediatric-adolescent subject, a pharmaceutical composition containing an anti-TNFα antibody or an antigen-binding fragment thereof.

For more ease of understanding the present invention, the terms used herein are defined below.

"TNFα" is intended to refer to a human cytokine which exists as a secreted form of 17 kD and a membrane-associated form of 26 kD, and of which a biologically active form is composed of a trimer non-covalently bound to a 17 kD molecule. The structure of TNFα is also described, for example, in Pennica, D., et al. (1984) Nature 312:724-729; Davis, J.M., et al. (1987) Biochemistry 26:1322-1326; and Jones, E.Y., et al. (1989) Nature 338:225-228.

An "antibody" refers to an immunoglobulin molecule composed of four polypeptide chains, in which two heavy chains and two light chains are linked to each other by disulfide bonds. Naturally occurring antibodies with other modified structures, e.g., camelid antibodies, are also included in this definition. Each heavy chain is composed of a heavy chain variable region and a heavy chain constant region. The heavy chain constant region is composed of three domains (CH1, CH2 and CH3). Each light chain is composed of a light chain variable region and a light chain constant region. The light chain constant region is composed of one domain (CL). The heavy chain variable region and the light chain variable region may be further subdivided into hypervariable regions called complementarity-determining regions (CDRs), arranged together with more conserved regions called framework regions (FRs). The heavy chain variable region and the light chain variable region are each composed of three CDRs and four FRs, which are arranged in the following order from the amino terminal to the carboxy terminal: FR1, CDR1, FR2, CDR2, FR3, CDR3, and FR4.

An "antigen-binding fragment" refers to one or more fragments of an antibody that has the ability to specifically bind to an antigen to which the whole antibody binds. Exemplary antigen-binding fragments include, but are not limited to, Fab, Fab', F(ab')2, Fv, and the like.

A "biosimilar" refers to a biological product that is highly similar to a biological product (reference product, reference drug) approved by a drug regulatory agency and has no clinically significant differences from the reference drug in terms of pharmacokinetics, safety, and efficacy.

A "biological formulation" or "biological product" refers to a pharmaceutical product which is prepared using a material derived from a human or other organism as a raw material or a substance, and which requires special attention in terms of health and hygiene, and includes a biological formulation, a genetically recombinant pharmaceutical product, a cell culture pharmaceutical product, a cell therapy, a gene therapy, and other formulations recognized by the Minister of Food and Drug Safety.

"Administration" refers to the administration of a substance (e.g., an anti-TNFα antibody) to achieve a therapeutic purpose (e.g., treating a TNFα-related disease).

"TNFα-related disease" refers to a local and/or systemic physiological disease in which TNFα is a major mediator that induces signs of the disease. The terms "TNFα-related disease," "disease that can be treated with anti-TNFα," and "disease in which TNFα activity is harmful" are used interchangeably herein.

A "subject" includes all human or non-human animals. The term "non-human animal" includes, but is not limited to, vertebrates such as non-human primates, sheep, dogs, cats, rabbits, and ferrets, rodents such as mice, rats, and guinea pigs, avian species such as chickens, amphibians, and reptiles. In preferred embodiments, the subject is a mammal such as a non-human primate, a sheep, a dog, a cat, a rabbit, a ferret, or a rodent. In more preferred embodiments, the subject is a human. The terms "subject," "patient," and "individual" are used interchangeably herein.

"IC50" is intended to refer to the concentration of an inhibitor required to inhibit a desired biological result, e.g., required to neutralize cytotoxic activity.

"C_{trough} (trough concentration)" or "minimum blood concentration" is the minimum blood concentration of a drug immediately before the next administration, and may refer to the minimum concentration of the drug in blood predicted using a population pharmacokinetic model.

"DAS28 (Disease activity score in 28 joints)" is a method for assessing the disease activity of rheumatoid arthritis (RA) based on 28 joints.

"CDAI (Crohn's disease activity index)" is a research tool used to quantify symptoms in subjects with Crohn's disease.

"PCDAI (Pediatric Crohn's disease activity index)" is a research tool used to quantify symptoms in pediatric-adolescent subjects with Crohn's disease.

"Mayo score" is a research tool used to quantify symptoms in subjects with ulcerative colitis.

A "disease-modifying anti-rheumatic drug (DMARD)" refers to a drug or a combination thereof that is effective in alleviating symptoms of arthritis and slowing the progression of the disease. The DMARD blocks an effect of releasing a chemical substance that causes an immune system to attack joints and damage bones, tendons, ligaments, and cartilage. Specific examples of DMARDs include methotrexate, hydroxychloroquine, sulfasalazine, and leflunomide.

A "kit" refers to a packaged product that includes components for administering the anti-TNFα antibody of the present invention for treatment of a TNFα-related disease. The kit preferably includes a container or box that retains the components of the kit. A protocol or label approved by the Food and Drug Administration is attached to the box or container. The components of the present invention are contained within a plastic, polyethylene, polypropylene, ethylene, or propylene box or container. The container may be a tube or bottle with a lid. The kit also includes instructions for the administration of the anti-TNFα antibody of the present invention.

Various aspects of the present invention are further described in detail herein.

### - An anti-TNFα antibody or an antigen-binding fragment thereof according to the present invention

In one embodiment of the present invention, the antibody may include a polyclonal antibody, monoclonal antibody, recombinant antibody, single-chain antibody, hybrid antibody, chimeric antibody, humanized antibody, or a fragment thereof. A chimeric antibody refers to an antibody including heavy chain and light chain variable region sequences from one species and constant region sequences from another species. In one embodiment of the present invention, the antibody may include a chimeric human-mouse IgG monoclonal antibody. The chimeric human-mouse IgG monoclonal antibody is composed of mouse heavy chain and light chain variable regions, and human heavy chain and light chain constant regions bound thereto. The chimeric human-mouse IgG monoclonal antibody may be produced by methods known in the art. For example, infliximab may be produced by the method disclosed in U.S. Patent No. 6,284,471.

In one embodiment of the present invention, the antibody may include an antibody that binds to TNFα or an epitope of TNFα. The antibody that binds to TNFα or an epitope of TNFα may include one or more selected from the group consisting of infliximab, adalimumab, certolizumab pegol, golimumab, and biosimilars thereof. In one embodiment of the present invention, the antibody may include infliximab. In this specification, infliximab may be expressed as CT-P13.

In one embodiment of the present invention, the antibody or antigen-binding fragment thereof may comprise: a light chain variable region comprising a CDR1 domain comprising an amino acid sequence of SEQ ID NO: 1, a CDR2 domain comprising an amino acid sequence represented by YAS, and a CDR3 domain containing an amino acid sequence of SEQ ID NO: 2; and a heavy chain variable region comprising a CDR1 domain comprising an amino acid sequence of SEQ ID NO: 3, a CDR2 domain comprising an amino acid sequence of SEQ ID NO: 4, and a CDR3 domain comprising an amino acid sequence of SEQ ID NO: 5.

In one embodiment of the present invention, the antibody or antigen-binding fragment thereof may comprise a light chain variable region comprising an amino acid sequence of SEQ ID NO: 6, and a heavy chain variable region comprising an amino acid sequence of SEQ ID NO: 7.

In one embodiment of the present invention, the antibody may comprise a light chain comprising an amino acid sequence of SEQ ID NO: 8, and a heavy chain comprising an amino acid sequence of SEQ ID NO: 9.

### -A composition containing an anti-TNFα antibody or an antigen-binding fragment thereof according to the present invention

The term a "composition containing an anti-TNFα antibody or an antigen-binding fragment thereof according to the present invention" herein is used interchangeably with a "stable liquid pharmaceutical formulation".

The composition according to the present invention may comprise (A) an antibody or an antigen-binding fragment thereof, (B) a surfactant, (C) a sugar or a derivative thereof, and (D) a buffer.

In the specification of this application, the term "does not comprise" means not comprising the component at all. Furthermore, the term encompasses the meaning of not substantially comprising the component, i.e., comprising the component within a range that does not affect the activity of an antibody, the stability and viscosity of a liquid pharmaceutical formulation, for example, comprising the component in an amount of 0 to 1% (w/v), 0 to 1 ppm (w/v), or 0 to 1 ppb (w/v) with respect to the total weight of the liquid pharmaceutical formulation.

### (A) Antibody or antigen-binding fragment thereof

In one embodiment, the composition according to the present invention may include the above-described anti-TNFα antibody or antigen-binding fragment thereof.

The concentration of the antibody or antigen-binding fragment thereof may be freely adjusted within a range that does not substantially adversely affect the stability and viscosity of the composition according to the present invention. In one embodiment of the present invention, the concentration of the antibody or antigen-binding fragment thereof may be 10 to 220 mg/ml. In another embodiment of the present invention, the concentration of the antibody or antigen-binding fragment thereof may be 50 to 220 mg/ml. In yet another embodiment of the present invention, the concentration of the antibody or antigen-binding fragment thereof may be 80 to 220 mg/ml. In still another embodiment of the present invention, the concentration of the antibody or antigen-binding fragment thereof may be 90 to 220 mg/ml. In yet another embodiment of the present invention, the concentration of the antibody or antigen-binding fragment thereof may be 90 to 180 mg/ml. In yet another embodiment of the present invention, the concentration of the antibody or antigen-binding fragment thereof may be 90 to 145 mg/ml. In yet another embodiment of the present invention, the concentration of the antibody or antigen-binding fragment thereof may be 110 to 130 mg/ml. When the concentration of the antibody or antigen-binding fragment thereof is within this range, the composition can exhibit excellent long-term stability and low viscosity while increasing the degree of freedom in administration dose and administration cycle due to the high content of the antibody or antigen-binding fragment thereof.

### (B) Surfactant

Examples of the surfactant include polyoxyethylene sorbitan fatty acid ester (e.g., polysorbate), polyoxyethylene alkyl ether (e.g., Brij), alkylphenyl polyoxyethylene ether (e.g., Triton-X), a polyoxyethylene-polyoxypropylene copolymer (e.g., Poloxamer, Pluronic), sodium dodecyl sulfate (SDS), and the like, but are not limited thereto.

In one embodiment of the present invention, the surfactant may include polyoxyethylene sorbitan fatty acid ester (polysorbate). The polysorbate may include polysorbate 20, polysorbate 40, polysorbate 60, polysorbate 80, or a mixture of two or more thereof. In one embodiment of the present invention, the polysorbate may include polysorbate 20, polysorbate 80, or a mixture thereof. In another embodiment of the present invention, the polysorbate may include polysorbate 80.

In one embodiment, the concentration of the surfactant may be freely adjusted within a range that does not adversely affect the stability and viscosity of the stable liquid pharmaceutical formulation according to the present invention. For example, the concentration of the surfactant may be 0.001 to 5% (w/v), 0.01 to 1% (w/v), or 0.02 to 0.1% (w/v). When the concentration of the surfactant is within this range, excellent long-term stability and low viscosity can be exhibited.

### (C) Sugar or sugar derivative

The sugar may include monosaccharides, disaccharides, oligosaccharides, polysaccharides, or mixtures of two or more thereof. Examples of monosaccharides include glucose, fructose, galactose, and the like, but are not limited thereto. Examples of disaccharides include sucrose, lactose, maltose, trehalose, and the like, but are not limited thereto. Examples of oligosaccharides include fructooligosaccharides, galactooligosaccharides, mannanooligosaccharides, and the like, but are not limited thereto. Examples of polysaccharides include starch, glycogen, cellulose, chitin, pectin, and the like, but are not limited thereto.

The sugar derivative may include sugar alcohols, sugar acids, or mixtures thereof. Examples of sugar alcohols include glycerol, erythritol, threitol, arabitol, xylitol, ribitol, mannitol, sorbitol, galactitol, fucitol, iditol, inositol, volemitol, isomalt, maltitol, lactitol, maltotriitol, maltotetraitol, polyglycitol, and the like, but are not limited thereto. Examples of sugar acids include aldonic acids (such as glyceric acid), ulosonic acids (such as neuraminic acid), uronic acids (such as glucuronic acid), and aldaric acids (such as tartaric acid), but are not limited thereto.

In one embodiment of the present invention, the sugar or the derivative thereof may include sorbitol, mannitol, trehalose, sucrose, or a mixture of two or more thereof.

In one embodiment of the present invention, the concentration of the sugar or the derivative thereof may be freely adjusted within a range that does not substantially adversely affect the stability and viscosity of the liquid pharmaceutical formulation according to the present invention. For example, the concentration of the sugar or the derivative thereof may be 0.1 to 30% (w/v), from 1 to 20% (w/v), or 1 to 10% (w/v). When the concentration of the sugar or the derivative thereof is within this range, long-term stability and low viscosity can be exhibited.

### (D) Buffer

The buffer is a neutralizing substance that minimizes changes in pH caused by acids or alkalis. Examples of the buffer include phosphate, acetate, succinate, gluconate, glutamate, citrate, histidine, and the like. In one embodiment of the present invention, the buffer may include acetate or histidine. When both acetate and histidine are included as the buffer, stability may be reduced.

In one embodiment of the present invention, the buffer may include acetate. Examples of the acetate include sodium acetate, zinc acetate, aluminum acetate, ammonium acetate, potassium acetate, and the like, but are not limited thereto. The buffer may additionally include an acid, such as acetic acid, for pH adjustment. It is most desirable to include acetate as the buffer in terms of pH adjustment and stability.

In one embodiment of the present invention, the buffer may include histidine. When histidine is used as the buffer, histidine salts, such as histidine chloride, histidine acetate, histidine phosphate, and histidine sulfate, and the like, may be included. An acid, such as hydrochloric acid, acetic acid, phosphoric acid, sulfuric acid, and the like, may be included for pH adjustment.

In one embodiment of the present invention, the stable liquid pharmaceutical formulation may not comprise citrate (citrate salt), phosphate, or a mixture thereof.

In one embodiment of the present invention, the amount of the buffer (or an anion of the buffer) may be freely adjusted within a range that does not substantially adversely affect the stability and viscosity of the liquid pharmaceutical formulation according to the present invention. For example, the amount of the buffer or anions thereof may be 1 to 90 mM, 5 to 30 mM, or 10 to 25 mM. When the amount of the buffer or anions thereof is within this range, long-term stability and low viscosity can be exhibited.

### (E) pH

In one embodiment of the present invention, the pH of the stable liquid pharmaceutical formulation may be 4.0 to 5.5 or 4.7 to 5.3. When the pH is within this range, long-term stability and low viscosity can be exhibited. The pH may be adjusted using the buffer. In other words, when a certain amount of the buffer is comprised, the pH of the above range can be achieved without using a separate pH regulator. It may be difficult to achieve the pH of the above range when citrate, phosphate, or a mixture thereof is used as the buffer. When an acid (e.g., hydrochloric acid) or base (e.g., sodium hydroxide) is additionally comprised as a separate pH regulator, the stability of the antibody may be reduced.

### (F) Other Components

In one embodiment of the present invention, the stable liquid pharmaceutical formulation may not comprise aspartic acid, lysine, arginine, or a mixture thereof. When these amino acids are comprised, the formulation may be solidified. In one embodiment of the present invention, the stable liquid pharmaceutical formulation may comprise at least one of amino acids except for the aforementioned three amino acids. In this case, the amino acid may be comprised in an amount in a range of 5% (w/v), for example, in a range of 0.001 to 5% (w/v), in a range of 0.001 to 1% (w/v), in a range of 0.01 to 5% (w/v), in a range of 0.01 to 1% (w/v), in a range of 0.1 to 5% (w/v), or in a range of 0.1 to 1% (w/v).

In another embodiment of the present invention, the stable liquid pharmaceutical formulation may comprise taurine. In this case, the taurine may be comprised in an amount in a range of 5% (w/v), for example, in a range of 0.001 to 5% (w/v), in a range of 0.001 to 1% (w/v), in a range of 0.01 to 5% (w/v), in a range of 0.01 to 1% (w/v), in a range of 0.1 to 5% (w/v), or in a range of 0.1 to 1% (w/v).

In one embodiment of the present invention, the stable liquid pharmaceutical formulation may not comprise NaCl, KCl, NaF, KBr, NaBr, Na₂SO₄, NaSCN, K₂SO₄, and the like as a metal salt. In a case where these metal salts are comprised, precipitation may occur, the formulation may have a gelatinous form, and the stability thereof may be lowered.

In one embodiment of the present invention, the stable liquid pharmaceutical formulation may not comprise a chelating agent (e.g., EDTA). In a case where the chelating agent is comprised, the oxidation rate may increase.

In one embodiment of the present invention, the stable liquid pharmaceutical formulation may not comprise a preservative. Examples of the preservative include octadecyl dimethyl benzyl ammonium chloride, hexamethonium chloride, benzalkonium chloride, benzethonium chloride, phenol, butyl alcohol, benzyl alcohol, alkyl paraben, catechol, resorcinol, cyclohexanol, 3-pentanol, m-cresol, and the like. Including the preservative may not contribute to improving stability.

In one embodiment of the present invention, the stable liquid pharmaceutical formulation of the present invention may further comprise additives known in the art, within a range that does not substantially adversely affect the activity of the antibody, the stability of the formulation, and the low viscosity thereof. For example, the stable liquid pharmaceutical formulation may further comprise an aqueous carrier, an antioxidant, or a mixture of two or more thereof. The aqueous carrier is a carrier which is pharmaceutically acceptable (i.e., safe and non-toxic when administered to humans) and useful for the preparation of a liquid pharmaceutical formulation. Examples of the aqueous carrier include sterile water for injection (SWFI), bacteriostatic water for injection (BWFI), sterile saline solution, Ringer's solution, dextrose, and the like, but are not limited thereto. The antioxidant includes ascorbic acid, and the like, but is not limited thereto.

### (G) "Stable" liquid pharmaceutical formulation

The term "stable" in the "stable" liquid pharmaceutical formulation of the present invention means that the antibody according to the present invention substantially retains its physical stability and/or chemical stability and/or biological activity during production processes and/or during storage/keeping. Various analytical techniques known in the art may be used to measure the stability of the antibody.

The physical stability may be assessed by methods known in the art, which include measurement of the apparent attenuation of light (absorbance or optical density) of samples. Such measurement of light attenuation relates to the turbidity of the formulation. Furthermore, the content of high-molecular-weight components, the content of low-molecular-weight components, intact protein mass, number of insoluble foreign particles, and the like, can be measured for the physical stability.

The chemical stability may be assessed by detecting and quantifying chemically modified forms of the antibody. The chemical stability includes, for example, charge changes (e.g., resulting from deamidation or oxidation) that may be assessed by ion exchange chromatography. Charge variants (acidic or basic peaks), and the like, can be measured for the chemical stability.

The biological activity may be assessed by methods known in the art, and for example, the antigen-binding affinity may be measured by ELISA.

In one embodiment of the present invention, the liquid pharmaceutical formulation can be stable over a long period of time.

In one embodiment of the present invention, the term "stable" liquid pharmaceutical formulation refers to a liquid pharmaceutical formulation that satisfies at least one of the following:

### Turbidity

- A liquid pharmaceutical formulation in which the absorbance A₆₀₀ measured by a spectrophotometer after being stored for 4 weeks at a temperature of 40°C ± 2°C is 0 to 0.0300 or 0 to 0.0700;
- A liquid pharmaceutical formulation in which the absorbance A₆₀₀ measured by a spectrophotometer after being stored for 4 weeks at a temperature of 40 °C ± 2 °C, at a relative humidity of 75 ± 5%, and under a sealed condition is 0 to 0.0300 or 0 to 0.0700;

### Main component content (Main peak)

- A liquid pharmaceutical formulation in which the main component measured by SE-HPLC after being stored for 4 weeks at a temperature of 40 °C ± 2 °C is 98% to 100%;
- A liquid pharmaceutical formulation in which the main component measured by SE-HPLC after being stored for 4 weeks at a temperature of 40 °C ± 2 °C, at a relative humidity of 75 ± 5%, and under a sealed condition is 98% to 100%;

### High-molecular-weight components (peak with a retention time earlier than that of the main peak (intact IgG))

- A liquid pharmaceutical formulation in which the high-molecular-weight component measured by SE-HPLC after being stored for 12 months at a temperature of 5°C ± 3°C is 0 to 1.00%;
- A liquid pharmaceutical formulation in which the high-molecular-weight component measured by SE-HPLC after being stored for 12 months at a temperature of 5 °C ± 3 °C and under a sealed condition is 0% to 1.00%;

### Low-molecular-weight component (peak with a retention time later than that of the main peak (intact IgG))

- A liquid pharmaceutical formulation in which the low-molecular-weight component content measured by SE-HPLC after being stored for 12 months at a temperature of 5°C ± 3°C is 0 to 0.40%;
- A liquid pharmaceutical formulation in which the low-molecular-weight component measured by SE-HPLC after being stored for 12 months at a temperature of 5°C ± 3°C and under a sealed condition is 0 to 0.40%;

### Content of intact immunoglobulin G

- A liquid pharmaceutical formulation in which the content of intact immunoglobulin G (Intact IgG%) measured by non-reducing CE-SDS after being stored for 12 months at a temperature of 5 °C ± 3 °C is 94.0% to 100%;
- A liquid pharmaceutical formulation in which the content of intact immunoglobulin G (Intact IgG %) measured by non-reducing CE-SDS after being stored for 12 months at a temperature of 5 °C ± 3 °C and under a sealed condition is 94.0% to 100%;
- A liquid pharmaceutical formulation in which the content of intact immunoglobulin G (Intact IgG %) measured by non-reducing CE-SDS after being stored for 4 weeks at a temperature of 40 °C ± 2 °C is 94.0% to 100%;
- A liquid pharmaceutical formulation in which the content of intact immunoglobulin G (Intact IgG%) measured by non-reducing CE-SDS after being stored for 4 weeks at a temperature of 40°C ± 2°C, at a relative humidity of 75 ± 5%, and under a sealed condition is 94.0% to 100%;

### Content of intact heavy and light chains

- A liquid pharmaceutical formulation in which the content of intact heavy and light chains (Intact HC+LC%) measured by reducing CE-SDS after being stored for 12 months at a temperature of 5 °C ± 3 °C is 99.0% to 100%;
- A liquid pharmaceutical formulation in which the content of intact heavy and light chains (Intact HC+LC%) measured by reducing CE-SDS after being stored for 12 months at a temperature of 5°C ± 3°C and under a sealed condition is 99.0% to 100%;
- A liquid pharmaceutical formulation in which the content of intact heavy and light chains (Intact HC+LC%) measured by reducing CE-SDS after being stored for 4 weeks at a temperature of 40 °C ± 2 °C is 98.0% to 100%;
- A liquid pharmaceutical formulation in which the content of intact heavy and light chains (Intact HC+LC%) measured by reducing CE-SDS after being stored for 4 weeks at a temperature of 40°C ± 2°C, at a relative humidity of 75 ± 5%, and under a sealed condition is 98.0% to 100%;

### Number of insoluble foreign particles

- A liquid pharmaceutical formulation in which the number of insoluble foreign particles (10.00 µm ≤, < 400.00 µm) measured by HIAC after being stored for 12 months at a temperature of 5°C ± 3°C is 0 to 1,000;
- A liquid pharmaceutical formulation in which the number of insoluble foreign particles (10.00 µm ≤, < 400.00 µm) measured by HIAC after being stored for 12 months at a temperature of 5 °C ± 3 °C and under a sealed condition is 0 to 1,000;
- A liquid pharmaceutical formulation in which the number of insoluble foreign particles (1.00 µm ≤, < 100.00 µm) measured by MFI after being stored for 4 weeks at a temperature of 40 °C ± 2 °C is 0 to 30,000;
- A liquid pharmaceutical formulation in which the number of insoluble foreign particles (1.00 µm <, < 100.00 µm) measured by MFI after being stored for 4 weeks at a temperature of 40 °C ± 2 °C, at a relative humidity of 75 ± 5%, and under a sealed condition is 0 to 30,000;
- A liquid pharmaceutical formulation in which the number of insoluble foreign particles (10.00 µm ≤, < 100.00 µm) measured by MFI after being stored for 4 weeks at a temperature of 40 °C ± 2 °C is 0 to 200;
- A liquid pharmaceutical formulation in which the number of insoluble foreign particles (10.00 µm ≤, < 100.00 µm), measured by MFI after being stored for 4 weeks at a temperature of 40 °C ± 2 °C, at a relative humidity of 75 ± 5%, and under a sealed condition is 0 to 200;
- A liquid pharmaceutical formulation in which the number of insoluble foreign particles (10.00 µm ≤, < 100.00 µm) measured by MFI after being stored for 6 weeks at a temperature of 40 °C ± 2 °C is 0 to 500;
- A liquid pharmaceutical formulation in which the number of insoluble foreign particles (10.00 µm ≤, < 100.00 µm) measured by MFI after being stored for 6 weeks at a temperature of 40 °C ± 2 °C, at a relative humidity of 75 ± 5%, and under a sealed condition is 0 to 500;

### Oxidation rate

- A liquid pharmaceutical formulation in which the oxidation rate of heavy chains Met 255 measured by LC-MS after being stored for 4 weeks at a temperature of 40 °C ± 2 °C is 0% to 2.5%;
- A liquid pharmaceutical formulation in which the oxidation rate of heavy chains Met 255 measured by LC-MS after being stored for 4 weeks at a temperature of 40 °C ± 2 °C, at a relative humidity of 75 ± 5%, and under a sealed condition is 0% to 2.5%;

### Charge variant

- A liquid pharmaceutical formulation in which the acidic peak measured by IEC-HPLC after being stored for 4 weeks at a temperature of 40 °C ± 2 °C is 20% to 35%;
- A liquid pharmaceutical formulation in which the acidic peak measured by IEC-HPLC after being stored for 4 weeks at a temperature of 40 °C ± 2 °C, at a relative humidity of 75 ± 5%, and under a sealed condition is 20% to 35%;
- A liquid pharmaceutical formulation in which the basic peak measured by IEC-HPLC after being stored for 4 weeks at a temperature of 40°C ± 2°C is 33% to 40%;
- A liquid pharmaceutical formulation in which the basic peak measured by IEC-HPLC after being stored for 4 weeks at a temperature of 40°C ± 2°C, at a relative humidity of 75 ± 5%, and under a sealed condition is 33% to 40%;

### TNFα binding affinity

- A liquid pharmaceutical formulation in which the TNFα binding affinity measured by ELISA after being stored for 12 months at a temperature of 5 °C ± 3 °C is 80% to 120%; and
- A liquid pharmaceutical formulation in which the TNFα binding affinity measured by ELISA after being stored for 12 months at a temperature of 5 °C ± 3 °C and under a sealed condition is 80% to 120%.

In one embodiment of the present invention, the viscosity measured after 1 month at a temperature of 40 °C ± 2 °C may be 0.5 cP to 10.0 cP. In another embodiment of the present invention, the viscosity measured after 6 months at a temperature of 5 °C ± 3 °C may be 0.5 cP to 5.0 cP.

### (H) Method for producing stable liquid pharmaceutical formulation

The stable liquid pharmaceutical formulation of the present invention may be produced using known methods, without being limited to specific methods. For example, the liquid pharmaceutical formulation may be produced by adding a buffer to a solution containing a surfactant and a sugar or a derivative thereof to adjust the pH of the solution, and then adding an antibody to the mixed solution. Alternatively, a liquid pharmaceutical formulation may be produced by preparing a solution containing some excipients in the final step of a purification process and then adding the remaining components. For example, a liquid pharmaceutical formulation may be prepared by preparing a solution containing an antibody, a buffer, and a sugar or a derivative thereof in the final step of the purification process, and then adding a surfactant to the resultant solution.

Furthermore, the production of the formulation may or may not include a lyophilization process.

In a case where the lyophilization process is not included, for example, the liquid pharmaceutical formulation of the present invention may be filled into a sealed container immediately after being prepared and subjected to treatment such as sterilization.

In a case where the lyophilization process is included, the liquid pharmaceutical formulation according to the present invention may be produced by, for example: preparing and lyophilizing a liquid pharmaceutical formulation of the present invention, or preparing, lyophilizing, and storing/keeping the liquid pharmaceutical formulation of the present invention; and then supplementing or replacing components that have been removed or modified by lyophilization and/or storage/keeping. Further, the liquid pharmaceutical formulation according to the present invention may be produced by: lyophilizing only components of the liquid pharmaceutical formulation of the present invention excluding components that are likely to be removed or modified by lyophilization and/or storage/keeping; and then adding the components that were previously excluded.

The Korean Patent Application Nos. 10-2017-0081814 and 10-2018-0102233, which were filed by the present applicant, and are incorporated herein by reference in their entirety.

### - A method for treating a TNFα-related disease of the present invention

The present invention provides a method for treating a TNFα-related disease comprising subcutaneously administering to a subject, particularly a subject who is 20 years old or less, preferably a pediatric-adolescent subject, a pharmaceutical composition containing an anti-TNFα antibody or an antigen-binding fragment thereof.

In one embodiment of the present invention, the antibody may include one or more selected from the group consisting of infliximab, adalimumab, certolizumab pegol, golimumab, and biosimilars thereof.

In one embodiment of the present invention, the antibody may include infliximab.

In one embodiment of the present invention, the antibody may include a chimeric human-mouse IgG monoclonal antibody.

In one embodiment of the present invention, the antibody or antigen-binding fragment thereof may comprise: a light chain variable region including a CDR1 domain comprising an amino acid sequence of SEQ ID NO: 1, a CDR2 domain comprising an amino acid sequence represented by YAS, and a CDR3 domain comprising an amino acid sequence of SEQ ID NO: 2; and a heavy chain variable region including a CDR1 domain comprising an amino acid sequence of SEQ ID NO: 3, a CDR2 domain comprising an amino acid sequence of SEQ ID NO: 4, and a CDR3 domain comprising an amino acid sequence of SEQ ID NO: 5.

In one embodiment of the present invention, the antibody or antigen-binding fragment thereof may comprise: a light chain variable region comprising an amino acid sequence of SEQ ID NO: 6; and a heavy chain variable region comprisingan amino acid sequence of SEQ ID NO: 7.

In one embodiment of the present invention, the antibody may comprise: a light chain comprising an amino acid sequence of SEQ ID NO: 8; and a heavy chain comprising an amino acid sequence of SEQ ID NO: 9.

In one embodiment of the present invention, the concentration of the antibody or antigen-binding fragment thereof may be 10 to 220 mg/ml.

The present invention also provides a method for treating a TNFα-related disease comprising subcutaneously administering to a subject a composition including: (A) an anti-TNFα antibody or an antigen-binding fragment thereof; (B) a surfactant; (C) a sugar or a derivative thereof; and (D) a buffer.

In one embodiment of the present invention, the subject to whom the antibody or antigen-binding fragment thereof is administered may be a subject/patient with a TNFα-related disease who is 20 years old or less, 19 years old or less, 18 years old or less, 17 years old or less, 2 to 18 years old, 2 to 17 years old, or 6 to 17 years old. Furthermore, the subject/patient may be referred to as a pediatric-adolescent patient herein.

The above-mentioned anti-TNFα antibody or antigen-binding fragment thereof (A), the surfactant (B), the sugar or derivative thereof (C), and the buffer (D) are as described above.

In one embodiment of the present invention, the surfactant (B) may include polysorbate, poloxamer or mixtures thereof.

In one embodiment of the present invention, the surfactant (B) may include polysorbate 20, polysorbate 40, polysorbate 60, polysorbate 80, or mixtures of two or more thereof.

In one embodiment of the present invention, the surfactant (B) may include polysorbate 80.

In one embodiment of the present invention, the concentration of the surfactant (B) may be 0.02 to 0.1% (w/v).

In one embodiment of the present invention, the sugar (C) may include monosaccharides, disaccharides, oligosaccharides, polysaccharides, or mixtures of two or more thereof, and the derivative of the sugar may include sugar alcohols, sugar acids, or mixtures thereof.

In one embodiment of the present invention, the sugar or derivative thereof (C) may include sorbitol, mannitol, trehalose, sucrose, or mixtures of two or more thereof.

In one embodiment of the present invention, the concentration of the sugar or derivative thereof (C) may be 1 to 10% (w/v).

In one embodiment of the present invention, the buffer (D) may include acetate or histidine.

In one embodiment of the present invention, the content of the buffer (D) may be 1 to 90 mM.

In one embodiment of the present invention, the pH of the composition may be 4.0 to 5.5.

In one embodiment of the present invention, the composition may not comprise aspartic acid, lysine, arginine, or mixtures thereof.

In one embodiment of the present invention, the composition may not comprise NaCl, KCl, NaF, KBr, NaBr, Na₂SO₄, NaSCN, K₂SO₄, or mixtures thereof.

In one embodiment of the present invention, the composition may not comprise a chelating agent.

In one embodiment of the present invention, the composition may not comprise a preservative.

In one embodiment of the present invention, the composition may further comprise an aqueous carrier, an antioxidant, or mixtures of two or more thereof.

In one embodiment of the present invention, the composition may have a viscosity of 0.5 cP to 10.0 cP after 1 month at a temperature of 40°C ± 2°C or a viscosity of 0.5 cP to 5.0 cP after 6 months at a temperature of 5°C ± 3°C.

In one embodiment of the present invention, the composition may comprise: (A) an anti-TNFα antibody or an antigen-binding fragment thereof, in particular, an antibody or an antigen-binding fragment thereof, comprising a light chain variable region comprising an amino acid sequence of SEQ ID NO: 1, a CDR2 domain comprising an amino acid sequence represented by YAS, and a CDR3 domain comprising an amino acid sequence of SEQ ID NO: 2, and a heavy chain variable region comprising an amino acid sequence of SEQ ID NO: 3, a CDR2 domain comprising an amino acid sequence of SEQ ID NO: 4, and a CDR3 domain comprising an amino acid sequence of SEQ ID NO: 5; (B) a surfactant; (C) a sugar or a derivative thereof; and (D) a buffer containing acetate or histidine.

In one embodiment of the present invention, the composition may comprise: (A) 90 to 220 mg/ml or 90 to 180 mg/ml of the anti-TNFα antibody or antigen-binding fragment thereof, in particular, an antibody or an antigen-binding fragment thereof, comprising a light chain variable region comprising a CDR1 domain comprising an amino acid sequence of SEQ ID NO: 1, a CDR2 domain comprising an amino acid sequence represented by YAS, and a CDR3 domain comprising an amino acid sequence of SEQ ID NO: 2, and a heavy chain variable region comprising a CDR1 domain comprising an amino acid sequence of SEQ ID NO: 3, a CDR2 domain comprising an amino acid sequence of SEQ ID NO: 4, and a CDR3 domain comprising an amino acid sequence of SEQ ID NO: 5; (B) 0.02 to 0.1% (w/v) of a surfactant; (C) 1 to 10% (w/v) of a sugar or a derivative thereof; and (D) 1 to 90 mM or 1 to 50 mM of a buffer containing acetate or histidine.

In one embodiment of the present invention, the composition may comprise: (A) 90 to 220 mg/ml or 90 to 180 mg/ml of an anti-TNFα antibody or an antigen-binding fragment thereof, in particular, an antibody or an antigen-binding fragment thereof, comprising a light chain variable region comprising a CDR1 domain comprising an amino acid sequence of SEQ ID NO: 1, a CDR2 domain comprising an amino acid sequence represented by YAS, and a CDR3 domain comprising an amino acid sequence of SEQ ID NO: 2, and a heavy chain variable region comprising a CDR1 domain comprising an amino acid sequence of SEQ ID NO: 3, a CDR2 domain comprising an amino acid sequence of SEQ ID NO: 4, and a CDR3 domain comprising an amino acid sequence of SEQ ID NO: 5; (B) 0.02 to 0.1% (w/v) of polysorbate; (C) 1 to 10% (w/v) of sorbitol; and (D) 1 to 90 mM or 1 to 50 mM of a buffer containing acetate.

In one embodiment of the present invention, the composition may be subcutaneously administered.

In one embodiment of the present invention, the composition may not undergo, prior to use, a reconstitution step, a dilution step, or both thereof.

In one embodiment of the present invention, the composition may be filled in a sterile container or a pre-filled syringe.

In one embodiment of the present invention, immediately prior to administering the composition to a subject, a portion of the composition filled in a sterile container or a pre-filled syringe may be subdivided into an administration dose for use. In one embodiment of the present invention, the stable composition may be filled in a pre-filled syringe prior to use.

In one embodiment of the present invention, the composition may be included in an auto-injector or a syringe for subcutaneous administration prior to use.

### TNFα-related disease

In one embodiment of the present invention, the TNFα-related disease is selected from the group consisting of rheumatoid arthritis, ulcerative colitis, Crohn's disease, plaque psoriasis, psoriatic arthritis, ankylosing spondylitis, juvenile idiopathic arthritis, neonatal hemolytic disease, inflammatory bowel disease, multiple sclerosis, prevention of organ transplant rejection, non-Hodgkin's lymphoma, metastatic cancer, retinopathy of prematurity, ovarian cancer, gastric cancer, head and neck cancer, osteoporosis, paroxysmal nocturnal hemoglobinuria, invasive candidiasis, breast cancer, melanoma, chronic lymphocytic leukemia, acute myelogenous leukemia, renal cell carcinoma, colorectal cancer, asthma, nasopharyngeal carcinoma, hemorrhagic shock, Staphylococcus aureus infection, and follicular lymphoma.

In one embodiment of the present invention, the TNFα-related disease may be a disease that can be treated by intravenous administration of infliximab.

In one embodiment of the present invention, the TNFα-related disease may be rheumatoid arthritis, ulcerative colitis, Crohn's disease, plaque psoriasis, psoriatic arthritis, or ankylosing spondylitis, which can be treated by intravenous administration of infliximab.

In one embodiment of the present invention, the TNFα-related disease may be a disease that has onset at 20 years old or less, 19 years old or less, 18 years old or less, 17 years old or less, 2 to 18 years old, 2 to 17 years old, or 6 to 17 years old.

In one embodiment of the present invention, the subject to whom the anti-TNFα antibody is administered is a subject who has an insufficient response to a disease-modifying antirheumatic drug (DMARD) including methotrexate.

In one embodiment of the present invention, the subject to whom the anti-TNFα antibody is administered is a subject who has not been treated with methotrexate or another DMARD previously.

In one embodiment of the present invention, the subject to whom the anti-TNFα antibody is administered is a subject who exhibits an increase in a serological indicator related to severe axial symptoms and inflammation, and who does not exhibit an adequate response to conventional treatment.

In one embodiment of the present invention, the subject to whom the anti-TNFα antibody is administered is a subject who does not exhibit an adequate response to systemic therapy including methotrexate, cyclosporine, or psoralen ultraviolet therapy A (PUVA), or does not have tolerance to such therapy, or for whom such therapy is contraindicated.

In one embodiment of the present invention, the subject to whom the anti-TNFα antibody is administered a subject who does not exhibit an adequate response to a treatment with a corticosteroid, 6-mercaptopurine, azathioprine, or an immunosuppressant, or does not have tolerance to such treatment, or for whom such a treatment method is contraindicated.

In one embodiment of the present invention, the subject to whom the anti-TNFα antibody is administered is a subject who does not exhibit a response to a conventional treatment including an antibiotic, excretion, or an immunosuppressive treatment.

In one embodiment of the present invention, the subject to whom the anti-TNFα antibody is administered may be a subject having: (i) moderately to severely active Crohn's disease (CD) with a PCDAI score of greater than 30 points, ileocolonic Crohn's disease with a Simplified Endoscopic Activity Score for Crohn's Disease (SES-CD) of 6 points or more, ileocolonic Crohn's disease with a SES-CD of 4 points or more and at least one segment with an ulcer score, and/or Crohn's disease diagnosed by radiology, histology or endoscopy; and/or (ii) moderately to severely active ulcerative colitis (UC) having a modified Mayo score of 5 to 9 points and/or ulcerative colitis diagnosed by radiology, histology or endoscopy.

In one embodiment of the present invention, the subject to whom the anti-TNFα antibody is administered may be a subject who has received adequate treatment with primary nutrition therapy and/or a corticosteroid and/or an immunosuppressant for active Crohn's disease but has not responded, or does not have tolerance, or for whom a medical contraindication is contraindicated.

In one embodiment of the present invention, after subcutaneous administration, the subject may have any one or more of the following characteristics:
a) DAS28 (Disease activity score in 28 joints) being reduced by at least 2.0; or
b) CDAI (Crohn's disease activity index) being reduced by at least 70.

### Administration dose and administration cycle

In one embodiment of the present invention, the anti-TNFα antibody or antigen-binding fragment thereof may be administered at a dose of 10 to 300 mg. Specifically, the anti-TNFα antibody or antigen-binding fragment thereof may be subcutaneously administered at a dose of 10, 20, 30, 40, 50, 60, 70, 80, 90, 100, 110, 120, 130, 140, 150, 160, 170, 180, 190, 200, 210, 220, 230, 240, 250, 260, 270, 280, 290, or 300 mg.

In another embodiment of the present invention, the anti-TNFα antibody or antigen-binding fragment thereof may be subcutaneously administered at a dose of 40 to 180 mg or 90 to 180 mg. In yet another embodiment, the anti-TNFα antibody or antigen-binding fragment thereof may be subcutaneously administered at a dose of 40 to 300 mg or 90 to 300 mg. In yet another embodiment, the anti-TNFα antibody or antigen-binding fragment thereof may be subcutaneously administered at a dose of 120 to 240 mg.

In one embodiment, the anti-TNFα antibody or the binding fragment thereof may be subcutaneously administered at a dose of 40 to 120 mg, 40 to 100 mg, 80 to 100 mg, 110 to 130 mg, 170 to 190 mg, or 230 to 250 mg.

In one embodiment of the present invention, the anti-TNFα antibody or antigen-binding fragment thereof may be subcutaneously administered to a subject with rheumatoid arthritis at a dose of 40 to 120 mg, 40 to 190 mg, 80 to 190 mg, 90 to 180 mg, 110 to 130 mg, 90 mg, 120 mg, or 180 mg.

In one embodiment of the present invention, the anti-TNFα antibody or antigen-binding fragment thereof may be subcutaneously administered at a dose of 40 to 120 mg, 40 to 250 mg, 80 to 250 mg, 110 to 250 mg, 110 to 130 mg, 120 to 240 mg, 140 to 160 mg, 170 to 190 mg, 230 mg to 250 mg, 120 mg, 150 mg, 180 mg, or 240 mg to a subject with ulcerative colitis, Crohn's disease, plaque psoriasis, psoriatic arthritis, or ankylosing spondylitis.

In one embodiment of the present invention, the anti-TNFα antibody or antigen-binding fragment thereof may be subcutaneously administered: at a dose of 90 to 180 mg in a case where a body weight of the subject is less than 80 kg; or at a dose of 190 to 270 mg in a case where a body weight of the subject is 80 kg or more.

In one embodiment of the present invention, the anti-TNFα antibody or antigen-binding fragment thereof may be subcutaneously administered: at a dose of 60 to 120 mg in a case where a body weight of the subject is 40 kg or more and/or the subject is 20 years old or less, 19 years old or less, 18 years old or less, 17 years old or less, 2 years old to 18 years old, 2 years old to 17 years old, or 6 years old to 17 years old or a pediatric-adolescent patient; and at a dose of 40 to 60 mg in a case where a body weight of the subject is 40 kg or less and/or is the subject who is 20 years old or less, 19 years old or less, 18 years old or less, 17 years old or less, 2 years old to 18 years old, 2 years old to 17 years old, or 6 years old to 17 years old or a pediatric-adolescent patient.

In one embodiment of the present invention, the anti-TNFα antibody or antigen-binding fragment thereof may be subcutaneously administered at a dose of 40 to 120 mg in a case where a subject is 20 years old or less, 19 years old or less, 18 years old or less, 17 years old or less, 2 years old to 18 years old, 2 years old to 17 years old, or 6 years old to 17 years old. In one embodiment of the present invention, in a case where a condition of the subject is not improved or the therapeutic response is lost, the dose of the anti-TNFα antibody or antigen-binding fragment thereof may be increased. More specifically, the dose may be increased by 1.1 to 3 times, 1.1 to 2.5 times, 1.1 to 2.1 times, 1.5 to 2.1 times, 1.7 to 2.1 times, or 2 times.

In one embodiment of the present invention, in a case where a condition of a subject who is 20 years old or less, 19 years old or less, 18 years old or less, 17 years old or less, 2 years old to 18 years old, 2 years old to 17 years old, or 6 years old to 17 years old, or a pediatric-adolescent patient is not improved or the therapeutic response is lost, the dose may be increased by 1.5 to 2 times. Furthermore, in a case where a body weight of the subject is 40 kg or more, the dose of the anti-TNFα antibody or antigen-binding fragment thereof may be increased from 60 mg to 120 mg; and in a case where a body weight of the subject is 40 kg or less, the dose of the anti-TNFα antibody or antigen-binding fragment thereof may be increased from 40 mg to 60 mg.

The criterion for determining that a therapeutic response has been lost in Crohn's disease may be that the total CDAI score is 220 or more due to an increase of the CDAI score of a patient by 70 points or more. Furthermore, the criterion for determining that a therapeutic response has been lost in pediatric- adolescent patients with Crohn's disease who are 20 years old or less, 19 years old or less, 18 years old or less, 17 years old or less, 2 years old to 18 years old, 2 years old to 17 years old, or 6 years old to 17 years old may be that a PCDAI value is 30 or more and is increased by 15 points or more compared to a PCDAI at the time of administering the anti-TNFα antibody or antigen-binding fragment thereof 5 times.

In ulcerative colitis, the criterion for determining that a therapeutic response has been lost may be that a subject satisfies the following condition a) and satisfies at least one of b) or c) below:
a) a case where a bleeding subscore increased by 1 point or more from the lowest score with an actual value greater than 1 point; and
b) a case where a partial Mayo score increased by 2 points or more from the lowest score with an actual value of at least 4 points; or
c) a case where an endoscopic subscore increased by 1 point or more from the lowest score with an actual value greater than 1 point.

Furthermore, the criterion for determining that a therapeutic response has been lost in a pediatric-adolescent ulcerative colitis subject who is 20 years old or less, 19 years old or less, 18 years old or less, 17 years old or less, 2 years old to 18 years old, 2 years old to 17 years old, or 6 years old to 17 years old, is that a total score is 5 points or more compared to the modified Mayo score as measured at the time of administering the anti-TNFα antibody or antigen-binding fragment thereof 5 times, and an endoscopic score is 2 points or more.

In one embodiment of the present invention, in a case where a condition of a subject is not improved and the dose of the anti-TNFα antibody or antigen-binding fragment thereof is increased up to 240 mg, it may be preferable not to further increase the dose. If a subject administered with a dose of 240 mg is administered with a higher dose, liver damage caused by high-concentration drugs may occur.

In one embodiment of the present invention, administration of an increased dose of the anti-TNFα antibody or antigen-binding fragment thereof may be performed after 5 weeks, 10 weeks, 15 weeks, 20 weeks, 21 weeks, 22 weeks, 23 weeks, 24 weeks, 25 weeks, 26 weeks, 27 weeks, 28 weeks, 29 weeks, 30 weeks, 31 weeks, 32 weeks, 32 weeks, or 35 weeks. More preferably, the dose may be increased after 30 weeks. In a case where the dose is increased earlier than that time, a time for confirming the efficacy for the existing dose may be insufficient, and in a case where the dose is increased later than that time, there may be a side effect in which a condition of the subject is deteriorated.

In one embodiment of the present invention, the anti-TNFα antibody or antigen-binding fragment thereof may be subcutaneously administered at intervals of 1 to 8 weeks. Specifically, the anti-TNFα antibody or antigen-binding fragment thereof may be subcutaneously administered at intervals of 1 week, 1.5 weeks, 2 weeks, 2.5 weeks, 3 weeks, 3.5 weeks, 4 weeks, 4.5 weeks, 5 weeks, 5.5 weeks, 6 weeks, 6.5 weeks, 7 weeks, 7.5 weeks, or 8 weeks.

In another embodiment of the present invention, the anti-TNFα antibody or antigen-binding fragment thereof may be subcutaneously administered at intervals of 2 to 4 weeks, preferably at intervals of 2 weeks.

In one embodiment of the present invention, the method may be an administration method of subcutaneously administering the anti-TNFα antibody or antigen-binding fragment thereof to a subject, and then maintaining a trough blood concentration (C_{trough}; minimum concentration immediately before the next application) of the anti-TNFα antibody or antigen-binding fragment thereof at 0.01 µg/ml or more. More specifically, the method may be an administration method of maintaining the trough blood concentration at 0.01 to 50 µg/ml, 0.01 to 45 µg/ml, 0.01 to 40 µg/ml, 0.01 to 35 µg/ml, 0.01 to 30 µg/ml, 0.01 to 25 µg/ml, 0.01 to 20 µg/ml, 0.01 to 15 µg/ml, 0.01 to 10 µg/ml, 0.01 to 6 µg/ml, 0.1 to 6 µg/ml, 5 µg/ml, or 1 µg/ml.

In one embodiment of the present invention, in a case of a subject having rheumatoid arthritis, the method may be an administration method of subcutaneously administering to the subject and then maintaining the trough blood concentration (C_{trough}) of the anti-TNFα antibody or antigen-binding fragment thereof at 0.01 µg/ml or more, 0.01 to 50 µg/ml, 0.01 to 40 µg/ml, 0.01 to 30 µg/ml, 1 to 40 µg/ml, or 1 µg/ml or more. Preferably, the trough blood concentration (C_{trough}) of the anti-TNFα antibody or antigen-binding fragment thereof for a rheumatoid arthritis subject may be 1 µg/ml.

In one embodiment of the present invention, in a case of a subject having at least one disease selected from the group consisting of ulcerative colitis, Crohn's disease, plaque psoriasis, psoriatic arthritis, and ankylosing spondylitis, the method may be an administration method of subcutaneously administering to the subject and then maintaining the trough blood concentration (C_{trough}) of the anti-TNFα antibody or antigen-binding fragment thereof at 0.01 µg/ml or more, 0.01 to 60 µg/ml, 0.01 to 50 µg/ml, 0.01 to 45 µg/ml, 5 to 50 µg/ml, or 5 µg/ml or more.

Preferably, the trough blood concentration (C_{trough}) of the anti-TNFα antibody or antigen-binding fragment thereof for an IBD subject may be 5 µg/ml.

### Pre-administration

Prior to the subcutaneous administration step of the anti-TNFα antibody or antigen-binding fragment thereof, a step in which the anti-TNFα antibody or antigen-binding fragment thereof is intravenously administered may be included.

In one embodiment of the present invention, prior to subcutaneous administration step, the step in which the anti-TNFα antibody or antigen-binding fragment thereof is intravenously administered may be performed at least once or at least twice, or two or three times.

In one embodiment of the present invention, a) a subject having a rheumatoid arthritis may be intravenously administered with the anti-TNFα antibody or antigen-binding fragment thereof two times prior to the subcutaneous administration, and b) a subject having any one or more diseases selected from the group consisting of ulcerative colitis, Crohn's disease, plaque psoriasis, psoriatic arthritis, and ankylosing spondylitis may be intravenously administered with the anti-TNFα antibody or antigen-binding fragment thereof two or three times prior to the subcutaneous administration.

In one embodiment of the present invention, the subject may be intravenously administered with the anti-TNFα antibody or antigen-binding fragment thereof two times at 0-week and 2-week before the subcutaneous administration, or three times at 0-week, 2-week, and 6-week.

In one embodiment of the present invention, a) a subject having a rheumatoid arthritis may be intravenously administered with the anti-TNFα antibody or antigen-binding fragment thereof two times at 0-week and 2-week prior to the subcutaneous administration, and b) a subject having any one or more diseases selected from the group consisting of ulcerative colitis, Crohn's disease, plaque psoriasis, psoriatic arthritis, and ankylosing spondylitis may be intravenously administered with the anti-TNFα antibody or antigen-binding fragment thereof two times at 0-week and 2-week, or three times at 0-week, 2-week, and 6-week prior to the subcutaneous administration.

In one embodiment of the present invention, prior to the subcutaneous administration step, a step in which the anti-TNFα antibody or antigen-binding fragment thereof is intravenously administered at a dose of 1 to 10 mg/kg may be included. Specifically, a step in which a dose of 1, 2, 3, 4, 5, 6, 7, 8, 9, or 10 mg/kg is intravenously administered may be included.

In another embodiment of the present invention, prior to the subcutaneous administration step, a step in which the anti-TNFα antibody or antigen-binding fragment thereof is intravenously administered at a dose of 2 to 8 mg/kg may be included. In yet another embodiment of the present invention, prior to the subcutaneous administration step, a step in which the anti-TNFα antibody or antigen-binding fragment thereof is intravenously administered at a dose of 3 to 5 mg/kg may be included.

In one embodiment of the present invention, a) a subject having a rheumatoid arthritis may be intravenously administered with the anti-TNFα antibody or antigen-binding fragment thereof at a dose of 3 mg/kg per administration, and b) a subject having any one or more diseases selected from the group consisting of ulcerative colitis, Crohn's disease, plaque psoriasis, psoriatic arthritis, and ankylosing spondylitis may be intravenously administered with the anti-TNFα antibody or antigen-binding fragment thereof at a dose of 5 mg/kg per administration.

In one embodiment of the present invention, prior to the subcutaneous administration step, a step in which the anti-TNFα antibody or antigen-binding fragment thereof is intravenously administered may be included, and a time interval between the final intravenous administration and the first subcutaneous administration may be 1 to 8 weeks. Specifically, a step of administering the anti-TNFα antibody or antigen-binding fragment thereof at intervals of 1, 1.5, 2, 2.5, 3, 3.5, 4, 4.5, 5, 5.5, 6, 6.5, 7, 7.5, or 8 weeks may be included.

In another embodiment of the present invention, prior to the subcutaneous administration step, a step in which the anti-TNFα antibody or antigen-binding fragment thereof is intravenously administered may be included, and a time interval between the final intravenous administration and the first subcutaneous administration may be 2 to 8 weeks, 2 to 4 weeks, 4 weeks, preferably 2 weeks.

In another embodiment of the present invention, the timing of the first subcutaneous administration was set to minimize a possibility of occurrence of ADA which would occur due to a low blood infliximab concentration by making a pre-administration concentration level during a subcutaneous administration period become as close as possible to a blood concentration in a steady state. An optimal interval between the final intravenous administration and the first subcutaneous administration by considering the aforementioned condition was determined through a simulation performed based on a developed population PK model. As a result of the simulation, an average blood concentration at 10-week, which is after 2 to 4 weeks, more preferably after 4 weeks from the final intravenous administration, is most similar to an expected level of a pre-administration concentration in the steady state within a maintenance period of subcutaneous administration, and a low blood concentration fluctuation was also shown. Therefore, it is expected that setting the first subcutaneous administration at the 10-week makes it possible to reach an average pre-administration concentration level in the steady state expected during a test period most quickly.

In a case where the first subcutaneous administration is performed earlier than 2 weeks after the final intravenous administration, the blood concentration may be higher than the expected pre-administration concentration level in the steady-state during the maintenance period of subcutaneous administration. In a case where the first subcutaneous administration is performed later than 6 weeks after the final intravenous administration, the blood concentration may be lower than the expected pre-administration concentration level in the steady-state during the maintenance period of subcutaneous administration, and may reach an average pre-administration concentration level in the steady state expected during a test period relatively late compared to the case of proceeding the subcutaneous administration at 4-week (10- week).

In another embodiment of the present invention, the subject may be a subject who has been intravenously administered with the anti-TNFα antibody or antigen-binding fragment thereof two times at 0-week and 2-week, or three times at 0-week, 2-week, and 6-week, prior to subcutaneous administration.

### Combination Administration

Other biologic formulations or chemotherapeutic agents may be administered together with the anti-TNFα antibody or antigen-binding fragment thereof. The administration is performed simultaneously with, prior to, or subsequent to the administration of the anti-TNFα antibody or antigen-binding fragment thereof.

In one embodiment of the present invention, the biologic formulation administered in combination may include etanercept, infliximab, adalimumab, sertolizumab pegol, golimumab, or a combination thereof.

In one embodiment of the present invention, the chemotherapeutic agent administered in combination may include a disease-modifying antirheumatic drug (DMARD), a steroid, or an immunosuppressant.

In one embodiment of the present invention, the disease-modifying antirheumatic drug (DMARD) administered in combination may include methotrexate, leflunomide, sulfasalazine, hydroxychloroquine, or a combination thereof.

In one embodiment of the present invention, the steroid administered in combination may include corticosteroid, glucocorticoid, cortisol, mineralocorticoid, aldosterone, or a combination thereof.

In one embodiment of the present invention, the immunosuppressant administered in combination may include azathioprine, 6-mercaptopurine, cyclosporin A, tacrolimus, mycophenolic acid, bredinin, an mTOR inhibitor, an anti-lymphocyte antibody, or a combination thereof.

In one embodiment of the present invention, an antihistamine, hydrocortisone, paracetamol, and/or a non-sedating antihistamine may be administered in combination with the anti-TNFα antibody or antigen-binding fragment thereof.

### - Product

The present invention also provides a product including a composition containing the anti-TNFα antibody or antigen-binding fragment thereof, and a container accommodating the composition in a sealed state.

The composition containing the anti-TNFα antibody or antigen-binding fragment thereof is as described above.

In one embodiment of the present invention, the product may be a kit including the composition containing the anti-TNFα antibody or antigen-binding fragment thereof.

In one embodiment of the present invention, the product may further include an instruction providing a use method, a storage method, or both, of the composition containing the anti-TNFα antibody or binding-fragment thereof. The use method may include a treatment of a disease in which TNFα activity is harmful, and may include an administration route, an administration dose, and an administration timing.

In one embodiment of the present invention, the container may be formed from materials such as glass, polymer (plastic), metal, and the like, but is not limited thereto. In one embodiment of the present invention, the container is a sterile container, a bottle, a vial, a cartridge, a syringe (pre-filled syringe, auto-injector), or a tube, but is not limited thereto. In one embodiment of the present invention, the container may be a glass or polymer vial, or a glass or polymer pre-filled syringe.

The specific product forms of the vials, cartridges, pre-filled syringes, auto-injectors, and the like, and the methods of filling the stable liquid pharmaceutical formulation into the vials, cartridges, pre-filled syringes, auto-injectors, and the like are readily available or may be practiced by those of ordinary skill in the art to which the present invention pertains. For example, U.S. Patent Nos. 4,861,335 and 6,331,174 disclose specific product forms and filling methods for pre-filled syringes. For example, U.S. Patent Nos. 5,085,642 and 5,681,291 disclose specific product forms and assembly methods for auto-injectors. Commercially available products such as vials, cartridges, pre-filled syringes, and auto-injectors may be used as is, or products may be custom-made in consideration of the physical properties of the composition containing the anti-TNFα antibody or antigen-binding fragment thereof, an administration site, an administration dose, and the like.

In one embodiment of the present invention, the inside of the container may not be coated with silicone oil. When coated with the silicone oil, stability may be reduced. The container may be a single-dose or multi-dose container.

In one embodiment of the present invention, the product may include other tools needed from commercial and user perspectives, such as needles, syringes, and the like.

Hereinafter, the present invention will be described in detail by way of examples. The following examples are intended to illustrate the present invention and do not limit the scope of the present invention.

### Example 1. Evaluation of Efficacy and Safety of Subcutaneous Injection of Infliximab as Maintenance Therapy for Patients with Pediatric Crohn's Disease (CD)

### Example 1-1. Clinical protocol

This clinical trial is a randomized, double-blind, multi-center, phase 3 trial designed so as to evaluate the efficacy, PK, PD, and safety of infliximab SC.

This clinical trial is composed of three study periods including a screening period, a treatment period (induction phase, maintenance phase, and extension phase) and a close out visit.

**Screening period:** Screening is performed between -42 days and day 0 (up to 6 weeks) prior to first administration of infliximab IV (intravenous injection) to be administered during the induction phase.

Patients must satisfy all of the following criteria to be enrolled in this clinical trial.
* A patient who is male or female aged 6 years old to 17 years old
* A patient with moderately to severely active Crohn's disease (CD) having a pediatric Crohn's disease activity index (PCDAI) score of greater than 30 points
* A patient having a Simplified Endoscopic Activity Score for Crohn's Disease of 6 points or more in an ileocolonic Crohn's disease patient, or having a SES-CD of 4 points or more in an ileal or colonic Crohn's disease patient having an ulcer score in at least one segment
* A patient who has been diagnosed to have Crohn's disease at any time prior to the first administration of a trial drug via radiology, biopsy, or endoscopy
* A person who has received adequate treatment for active Crohn's disease with primary nutrition therapy and/or a corticosteroid and/or an immunosuppressant but has shown no response, intolerance, or who has medical contraindications thereto.

On the other hand, patients cannot be enrolled in the clinical trial if they meet any one of the following criteria:
* A patient who has previously used two or more biologic formulations, two or more Janus kinase (JAK) inhibitors, or a combination of two or more JAK inhibitors and two or more biologic formulations
* A patient who has used a TNF-α inhibitor or a biological formulation within 5 half-lives prior to the first administration of a clinical drug (day 0)
* A patient who has not responded to or was intolerant of a previously used TNF-α inhibitor for the treatment of Crohn's disease
* A patient who has a history of being administered with infliximab for the treatment of Crohn's disease or another disease
* A patient who is allergic to infliximab or other murine and/or human protein excipients, or who is hypersensitive to immunoglobulin products
* A patient who has received a diagnosis of very early onset inflammatory bowel disease with evidence or a clinical feature of a basic genetic defect that affects immune function or impairs epithelial barrier function (monogenic inflammatory bowel disease).

**Composition and Administration Method of Trial Drug to be Subcutaneously Administered:** A trial drug administered to a patient is a composition containing infliximab, sorbitol, polysorbate, acetic acid, and water for injection. The trial drug is a formulation in which 120 mg of infliximab, sorbitol, polysorbate, acetic acid, and water for injection are filled in a pre-filled syringe and which is marketed as Remsima SC in Europe, and in a case where a dose is 40 mg or 60 mg, a portion of the trial drug included in the pre-filled syringe may be dispensed into a sterile vial and then subcutaneously administered to a patient through a syringe for subcutaneous administration.

### Treatment period:

* Open-label induction phase (administered at 0-week, 2-week, and 6-week)
* Double-blind maintenance phase (administered from 10-week to 54-week)
* Double-blind extension phase (administered from 56-week to 102-week)

In the open-label induction phase, only a patient who satisfies all selection criteria and does not satisfy any exclusion criteria as of day 0 (0-week) is enrolled in the clinical trial. All enrolled patients visit at 0-week, 2-week, and 6-week for induction therapy and are administered with infliximab IV (5 mg/kg) for 2 hours. Among patients who have been administered with a full dose of infliximab three times via IV infusion, a patient having a PCDAI score of 30 points or less at 10-week is classified as a responder, and only patients having no safety concerns are randomly assigned before administration on day 70 (10-week) to be administered with either high-dose infliximab or low-dose infliximab via an SC route, at the discretion of an investigator.

Randomization for administration assignment is stratified by the following criteria:
* Body weight at 10-week (less than 40 kg or 40 kg or more)
* Experience with use of a biological formulation and/or a JAK inhibitor (use or not use)
* Achievement of clinical remission at 10-week (remission achieved or not achieved by a PCDAI score)

The double-blind maintenance phase is composed of administration of an additional dose of high-dose infliximab or low-dose infliximab via an SC route, and the last dose is administered at 54-week.
* High-dose group 1)
   Administration of infliximab 120 mg via the SC route every 2 weeks from 10-week to 54-week (body weight of a patient ≥ 40 kg)
   Administration of infliximab 60 mg via the SC route every 2 weeks from 10-week to 54-week (body weight of a patient < 40 kg)
* Low-dose group 2)
   Administration of infliximab 60 mg via the SC route every 2 weeks from 10-week to 54-week (body weight of a patient≥ 40 kg)
   Administration of infliximab 40 mg via the SC route every 2 weeks from 10-week to 54-week (body weight of a patient < 40 kg)

In the double-blind extension phase, a patient who has completed the maintenance phase up to 54-week and who is deemed to benefit from continued treatment at the discretion of an investigator receives the same dose as in the maintenance phase, and the extension phase begins at 56-week and continues until 102-week.

In a case where a patient in the low-dose group initially responds to a drug but subsequently loses that response, an increase to a high dose is allowed starting at 22-week. Loss of response is defined as a case where a PCDAI score is 30 points or higher, and is increased by 15 points or more compared to that in 10-week.

Only in the double-blind extension phase, if the body weight of a patient increases from < 40 kg to ≥ 40 kg, a dose increase is permitted from 40 mg or 60 mg of infliximab to 60 mg or 120 mg of infliximab.

A patient may receive premedication 30 to 60 minutes before infliximab IV administration, and without limitation thereto, antihistamine, hydrocortisone, paracetamol, and/or non-sedating antihistamine may be administered at the discretion of an investigator in the clinical period. A patient may also receive premedication at the discretion of an investigator in a case of infliximab SC administration.

**Close Out Visit:** A last visit for ending the clinical trial is conducted 4 weeks after the last administration. For a patient who discontinues study treatment early before switching to administration of the high-dose or low-dose infliximab via an SC route at 10-week, the close out visit is conducted 8 weeks after the last administration of infliximab IV.

### Example 1-2. Population PK Analysis Results and PK-PD Modeling for Pediatric-Adolescent CD Patients

A population PK analysis result and PK-PD modeling for pediatric-adolescent CD patients were based on data from intravenous administration of infliximab to healthy adult subjects, adult patients with ankylosing spondylitis, adult patients with rheumatoid arthritis, and adult patients with Crohn's disease, and data from infliximab subcutaneous administration in healthy adult subjects, adult patients with rheumatoid arthritis, adult patients with Crohn's disease, and adult patients with ulcerative colitis (see Clinicaltrials.gov identifier numbers NCT01220518, NCT01217086, NCT02096861, NCT03147248, NCT02883452, NCT04205643, and NCT03945019).

Population PK-PD modeling for pediatric-adolescent CD patients was performed by stratifying virtual patients representing pediatric-adolescent patients aged 2 years old to less than 18 years old into weight groups of 10 kg units, and exploring maintenance doses of 40, 60, 80, 100, and 120 mg SC Q2W (from 10-week) in a virtual pediatric-adolescent patient population and comparing with exposure values achieved according to the 120 mg SC Q2W (from 10-week) maintenance dose regimen in an adult patient group.

A simulated CDAI result showed no differences across administration doses and body weights, and an infliximab administration dose was selected based on the PK simulation result.

The PK-PD model constructed based on the above data can be used to simulate subcutaneous administration results for pediatric-adolescent patients with indications for infliximab (e.g., rheumatoid arthritis, ulcerative colitis, Crohn's disease, plaque psoriasis, psoriatic arthritis, or ankylosing spondylitis).

A final population PK model was performed as a two-compartment model with linear elimination from a central compartment where infliximab infusion occurs and a depot compartment with a primary absorption rate to the central compartment. A covariate relationship between disease duration and a baseline CDAI score was applied to the model. The PK-PD modeling validation was performed through prediction corrected visual predictive checks (pcVPC).

### Example 1-3. Simulation Result for Determination of Doses for Pediatric-Adolescent CD Patients

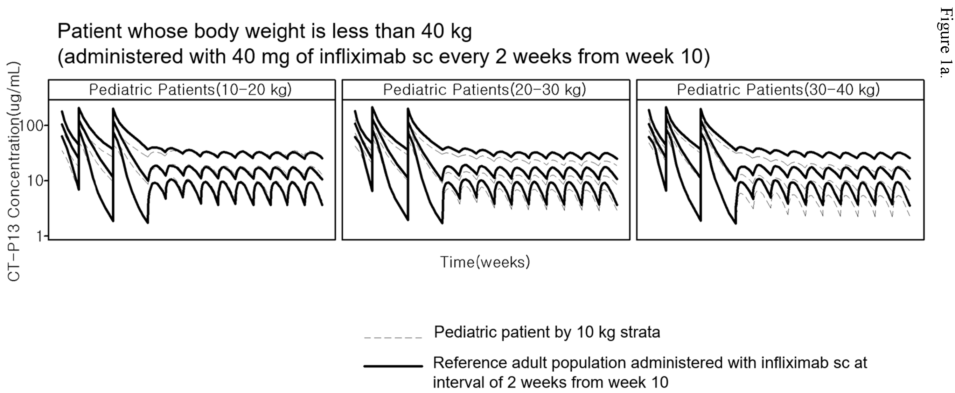

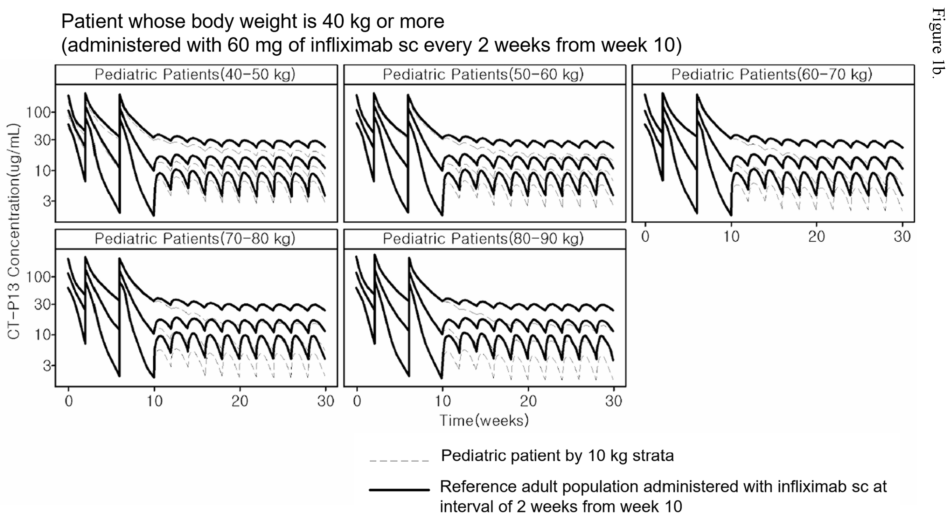

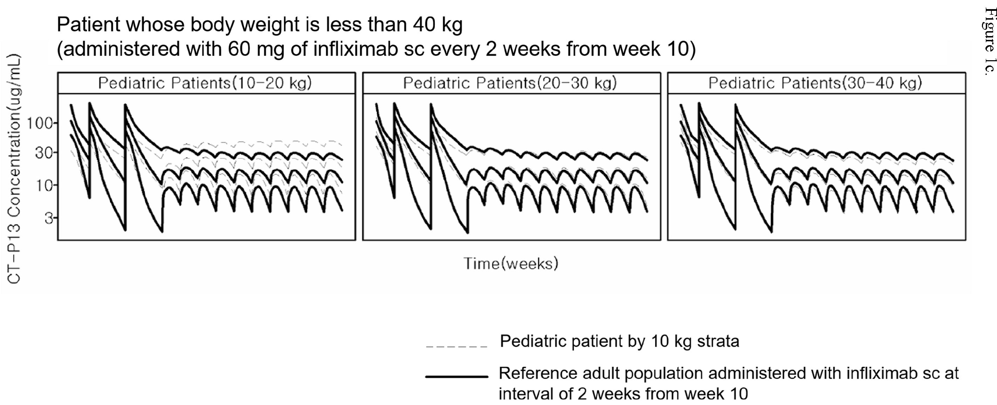

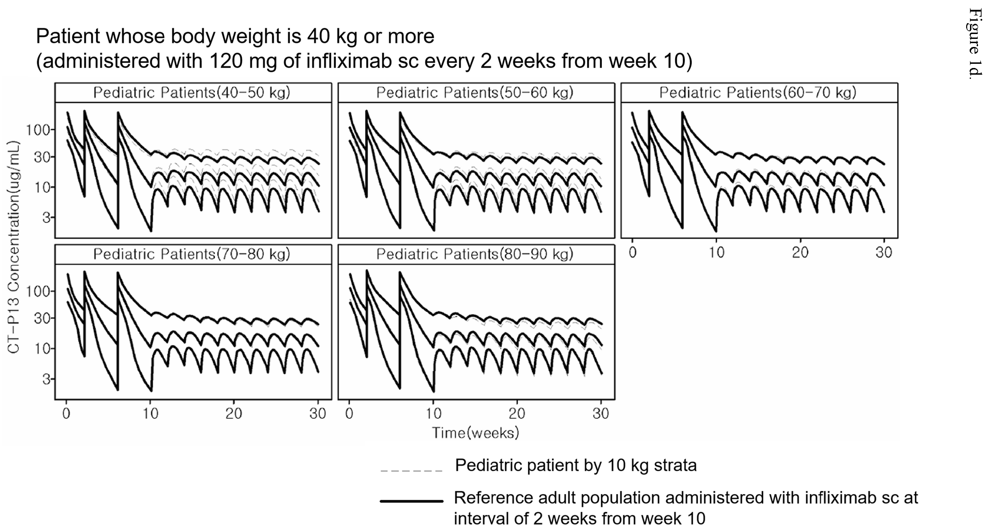

As a result of a simulation of a PK parameter by body weight strata, doses of 120 mg, 60 mg, and 40 mg were found to be within a PK equivalence margin of 80% to 125% (see Tables 1 to 3). Furthermore, a simulated PK profile for a low-dose and high-dose group is shown in Fig. 1. In this simulation, since higher drug exposure was observed in a group in which a pediatric-adolescent body weight is 10-20 kg (60 mg infliximab Q2W maintenance dose) and a group in which a pediatric-adolescent body weight is 40-60 kg (120 mg infliximab Q2W maintenance dose) than in an adult group (120 mg Q2W maintenance dose), an additional PK simulation was conducted. Accordingly, when compared to a drug exposure at a 240 mg infliximab Q2W maintenance dose in an adult patient population of NCT02883452, NCT04205643 and NCT03945019, it was shown that administering 60 mg or 120 mg every 2 weeks to a pediatric-adolescent patient (high-dose group) resulted in lower drug exposure in AUC and Cₘₐₓ. Since no safety issue was observed in a clinical trial result of an adult patient population administered with a 240 mg infliximab Q2W maintenance dose, the administration dose mentioned in Example 1-1 is predicted to be an appropriate dose in terms of safety.

Based on this result, an administration method for a pediatric-adolescent CD patient below is as follows so as to be exposed to a minimum drug while showing a maximum efficacy:
* High-dose group
   Subcutaneous administration of 120 mg of infliximab every 2 weeks from 10-week to 54-week (body weight of a patient ≥ 40 kg)
   Subcutaneous administration of 60 mg of infliximab every 2 weeks from 10-week to 54-week (body weight of a patient < 40 kg)
* Low-dose group
   Subcutaneous administration of 60 mg of infliximab every 2 weeks from 10-week to 54-week (body weight of a patient ≥ 40 kg)
   Subcutaneous administration of 40 mg of infliximab every 2 weeks from 10-week to 54-week (body weight of a patient < 40 kg)

**[Table 1]**

| **Geometric mean ratio and 90% confidence interval of simulated C_{trough,w30} of selected administration method** | | | |
|---|---|---|---|
| **Administration method** | **Test population** | **Geometric mean ratio** | **90% confidence interval** |
| **High-dose group** | | | |
| Patient of 40 kg or more SC administered with 120 mg of infliximab every 2 weeks | 80 - 90 kg | 0.87 | (0.83, 0.91) |
| | 70 - 80 kg | 0.96 | (0.92, 1.00) |
| | 60 - 70 kg | 1.08 | (1.03, 1.12) |
| | 50 - 60 kg | 1.23 | (1.18, 1.28) |
| | 40 - 50 kg | 1.44 | (1.38, 1.51) |
| Patient less than 40 kg SC administered with 60 mg of infliximab every 2 weeks | 30 - 40 kg | 0.88 | (0.85, 0.92) |
| | 20 - 30 kg | 1.16 | (1.11, 1.21) |
| | 10 - 20 kg | 1.75 | (1.68, 1.83) |

| **Low-dose group** | | | |
|---|---|---|---|
| Patient of 40 kg or more SC administered with 60 mg of infliximab every 2 weeks | 80 - 90 kg | 0.44 | (0.42, 0.45) |
| | 70 - 80 kg | 0.48 | (0.46, 0.50) |
| | 60 - 70 kg | 0.54 | (0.52, 0.56) |
| | 50 - 60 kg | 0.62 | (0.59, 0.64) |
| | 40 - 50 kg | 0.72 | (0.69, 0.75) |
| Patient less than 40 kg SC administered with 40 mg of infliximab every 2 weeks | 30 - 40 kg | 0.59 | (0.57, 0.62) |
| | 20 - 30 kg | 0.77 | (0.74, 0.81) |
| | 10 - 20 kg | 1.17 | (1.12, 1.22) |

| | | | |
|---|---|---|---|
| * In a simulated adult administration method, infliximab IV (5 mg/kg) was intravenously administered for 2 hours or more at 0-week, 2-week, and 6-week, and then, 120 mg of infliximab was subcutaneously administered at intervals of 2 weeks from 10-week. * In a simulated pediatric-adolescent administration method, infliximab IV (5 mg/kg) was intravenously administered for 2 hours or more at 0-week, 2-week, and 6-week, and then, 40 mg, 60 mg, 80 mg, 100 mg, or 120 mg of infliximab was subcutaneously administered at intervals of 2 weeks from 10-week | | | |

**[Table 2]**

| **Geometric mean ratio and 90% confidence interval of simulated C_{max,w28} of selected administration method** | | | |
|---|---|---|---|
| **Administration method** | **Test population** | **Geometric mean ratio** | **90% confidence interval** |
| **High-dose group** | | | |
| Patient of 40 kg or more SC administered with 120 mg of infliximab every 2 weeks | 80 - 90 kg | 0.88 | (0.85, 0.90) |
| | 70 - 80 kg | 0.96 | (0.94, 0.99) |
| | 60 - 70 kg | 1.07 | (1.04, 1.10) |
| | 50 - 60 kg | 1.21 | (1.18, 1.24) |
| | 40 - 50 kg | 1.40 | (1.37, 1.44) |
| Patient less than 40 kg SC administered with 60 mg of infliximab every 2 weeks | 30 - 40 kg | 0.85 | (0.82, 0.87) |
| | 20 - 30 kg | 1.09 | (1.06, 1.12) |
| | 10 - 20 kg | 1.60 | (1.56, 1.65) |

| **Low-dose group** | | | |
|---|---|---|---|
| Patient of 40 kg or more SC administered with 60 mg of infliximab every 2 weeks | 80 - 90 kg | 0.44 | (0.43, 0.45) |
| | 70 - 80 kg | 0.48 | (0.47, 0.50) |
| | 60 - 70 kg | 0.54 | (0.52, 0.55) |
| | 50 - 60 kg | 0.61 | (0.59, 0.62) |
| | 40 - 50 kg | 0.70 | (0.68, 0.72) |
| Patient less than 40 kg SC administered with 40 mg of infliximab every 2 weeks | 30 - 40 kg | 0.57 | (0.55, 0.58) |
| | 20 - 30 kg | 0.73 | (0.71, 0.75) |
| | 10 - 20 kg | 1.07 | (1.04, 1.10) |

| | | | |
|---|---|---|---|
| * In a simulated adult administration method, infliximab IV (5 mg/kg) was intravenously administered for 2 hours or more at 0-week, 2-week, and 6-week, and then, 120 mg of infliximab was subcutaneously administered at intervals of 2 weeks from 10-week. * In a simulated pediatric-adolescent administration method, infliximab IV (5 mg/kg) was intravenously administered for 2 hours or more at 0-week, 2-week, and 6-week, and then, 40 mg, 60 mg, 80 mg, 100 mg, or 120 mg of infliximab was subcutaneously administered at intervals of 2 weeks from 10-week. | | | |

**[Table 3]**

| **Geometric mean ratio and 90% confidence interval of simulated AUC_{w28-30} of a selected administration method** | | | |
|---|---|---|---|
| **Administration method** | **Test population** | **Geometric mean ratio** | **90% confidence interval** |
| **High-dose group** | | | |
| Patient of 40 kg or more SC administered with 120 mg of infliximab every 2 weeks | 80 - 90 kg | 0.87 | (0.85, 0.90) |
| | 70 - 80 kg | 0.96 | (0.93, 0.99) |
| | 60 - 70 kg | 1.07 | (1.04, 1.11) |
| | 50 - 60 kg | 1.22 | (1.18, 1.26) |
| | 40 - 50 kg | 1.42 | (1.37, 1.46) |
| Patient less than 40 kg SC administered with 60 mg of infliximab every 2 weeks | 30 - 40 kg | 0.86 | (0.83, 0.89) |
| | 20 - 30 kg | 1.11 | (1.08, 1.15) |
| | 10 - 20 kg | 1.65 | (1.60, 1.70) |

| **Low-dose group** | | | |
|---|---|---|---|
| Patient of 40 kg or more SC administered with 60 mg of infliximab every 2 weeks | 80 - 90 kg | 0.44 | (0.42, 0.45) |
| | 70 - 80 kg | 0.48 | (0.47, 0.50) |
| | 60 - 70 kg | 0.54 | (0.52, 0.55) |
| | 50 - 60 kg | 0.61 | (0.59, 0.63) |
| | 40 - 50 kg | 0.71 | (0.69, 0.73) |
| Patient less than 40 kg SC administered with 40 mg of infliximab every 2 weeks | 30 - 40 kg | 0.57 | (0.56, 0.59) |
| | 20 - 30 kg | 0.74 | (0.72, 0.77) |
| | 10 - 20 kg | 1.10 | (1.07, 1.14) |

| | | | |
|---|---|---|---|
| * In a simulated pediatric-adolescent administration method, infliximab IV (5 mg/kg) was intravenously administered for 2 hours or more at 0-week, 2-week, and 6-week , and then, 40 mg, 60 mg, 80 mg, 100 mg, or 120 mg of infliximab was subcutaneously administered at intervals of 2 weeks from 10-week. | | | |

### Example 2. Evaluation of Efficacy and Safety of Subcutaneous Injection of Infliximab as Maintenance Therapy for Pediatric-Adolescent Patients With Ulcerative Colitis (UC)

### Example 2-1. Clinical protocol

This clinical trial is a randomized, double-blind, multi-center, phase 3 trial designed to evaluate the efficacy, PK, PD, and safety of infliximab SC.

This clinical trial is composed of three study periods including a screening period, a treatment period (induction phase, maintenance phase, and extension phase), and a close out visit.

**Screening period:** Screening is performed between -42 days and day 0 (up to 6 weeks) prior to first administration of infliximab IV administered during the induction phase.

Patients must satisfy all of the following criteria to be enrolled in this clinical trial.
* A patient who is male or female aged 6 years old to 17 years old
* A patient with moderately to severely active ulcerative colitis (UC) having a modified Mayo score of 5 to 9 points
* A patient who has been diagnosed with ulcerative colitis through endoscopy or radiology and histological examination (a histopathology report supporting a diagnosis must be available in a source document, prior to the first administration of the clinical drug (day 0))
* A patient who has received treatment for active ulcerative colitis but has not responded to, is intolerant to, or has medical contraindication to a conventional therapeutic agent such as a corticosteroid and/or 6-mercaptopurine or azathioprine

On the other hand, patients cannot be enrolled in the clinical trial, if they meet any one of the following criteria:
* A patient who has previously used two or more biologic formulations, or used two or more JAK inhibitors, or a combination of two or more JAK inhibitors and two or more biologic formulations
* A patient who has used a TNF-α inhibitor or a biologic formulation within 5 half-lives prior to the first administration of a clinical drug (day 0)
* A patient who has previously been administered with a TNF-α inhibitor for treatment of UC but did not show a response or was intolerant
* A patient who has a history of being administered with infliximab for the treatment of UC or another disease
* A patient who is allergic to infliximab or other murine and/or human protein excipients, or who is hypersensitive to immunoglobulin products
* A patient who has received a diagnosis of very early onset inflammatory bowel disease with evidence or a clinical feature of an underlying genetic defect that affects immune function or impairs epithelial barrier function (monogenic inflammatory bowel disease)

### Composition and Administration Method of Subcutaneously Administered Trial Drug:

A trial drug administered to a patient is a composition containing infliximab, sorbitol, polysorbate, acetic acid, and water for injection. The trial drug is a formulation in which 120mg of infliximab, sorbitol, polysorbate, acetic acid, and water for injection are filled in a pre-filled syringe and which is marketed as Remsima SC in Europe, and in a case where a dose is 40 mg or 60 mg, a portion of the trial drug included in the pre-filled syringe may be subdivided and transferred to a sterile vial and then subcutaneously administered to a patient through a syringe for subcutaneous administration.

### Treatment period:

* Open-label induction phase (administered at 0-week, 2-week, and 6-week)
* Double-blind maintenance phase (administered from 10-week to 54-week)
* Double-blind extension phase (administered from 56-week to 102-week)

In the open-label induction phase, only a patient who satisfies all selection criteria and does not satisfy any exclusion criteria as of day 0 (0- week) is enrolled in the clinical trial. All enrolled patients visit at 0-week, 2-week, and 6-week for induction therapy and are administered with infliximab IV (5 mg/kg) for 2 hours. Among patients classified as responders through a Mayo score at 10-week among patients who have been administered with a full dose of infliximab three times via IV infusion, only patients who are deemed to have no safety concerns at the discretion of an investigator, are randomly assigned before administration on day 70 (10-week) to be administered with either high-dose infliximab or low-dose infliximab via an SC route.

Randomization for administration assignment is stratified by the following criteria:
- Body weight at 10-week (< 40 kg or ≥ 40 kg)
- Experience with use of a biological formulation and/or a JAK inhibitor (use or not use)
- Achievement of clinical remission at 10-week (remission achieved or not achieved by a modified Mayo score)

The double-blind maintenance phase is composed of administration of an additional dose of high-dose infliximab or low-dose infliximab via an SC route, and the last dose is administered at 54-week.
* High-dose group 1)
   - Administration of infliximab 120 mg via the SC route every 2 weeks from 10-week to 54-week (body weight of a patient ≥40 kg)
   - Administration of infliximab 60 mg via the SC route every 2 weeks from 10-week to 54-week (body weight of a patient < 40 kg)
* Low-dose group 2)
   - Administration of infliximab 60 mg via the SC route every 2 weeks from 10-week to 54-week (body weight of a patient ≥ 40 kg)
   - Administration of infliximab 40 mg via the SC route every 2 weeks from 10-week to 54-week ((body weight of a patient <40 kg)

In the double-blind extension phase, a patient who has completed the maintenance phase up to 54-week and who is deemed to benefit from continued treatment at the discretion of an investigator receives the same dose as in the maintenance phase, and the extension phase begins at 56-week and continues until 102-week.

In a case where a patient in the low-dose group initially responds to a drug but subsequently loses that response, an increase to a high-dose is allowed starting at 22-week. Loss of response is defined as any one of the following: a case where a modified Mayo score is increased by 2 points or more and 30% or more compared to 10-week, and a total score is 5 points or more, and an endoscopic score is 2 points or more.

Only in the double-blind extension phase, if the body weight of a patient increases from <40 kg to ≥40 kg, a dose increase is permitted from infliximab 40 mg or 60 mg of infliximab to 60 mg or 120 mg of infliximab.

A patient may receive premedication 30 to 60 minutes before infliximab IV administration, and without limitation thereto, antihistamine, hydrocortisone, paracetamol, and/or a non-sedating antihistamine may be administered at the discretion of an investigator in the clinical period. A patient may also receive premedication at the investigator's discretion in a case of infliximab SC administration.

**Close Out Visit:** A last visit for ending the clinical trial is conducted 4 weeks after the last administration. For a patient who discontinues study treatment early before switching to administration of the high-dose or low-dose infliximab via an SC route at 10-week, the close out visit is conducted 8 weeks after the last infliximab IV administration.

### Example 2-2. Population PK Analysis Result and PK-PD Modeling for Pediatric-Adolescent UC Patients

A population PK analysis result and PK-PD modeling were based on data from intravenous administration of infliximab to healthy adult subjects, adult patients with ankylosing spondylitis, adult patients with rheumatoid arthritis, and an adult patient with Crohn's disease, and data from subcutaneous administration of infliximab to healthy adult subjects, adult patients with rheumatoid arthritis, an adult patients with Crohn's disease, and an adult patients with ulcerative colitis (see Clinicaltrials.gov identifier numbers NCT01220518, NCT01217086, NCT02096861, NCT03147248, NCT02883452, NCT04205643, and NCT03945019).

Population PK analysis result and PK-PD modeling for pediatric-adolescent UC patients were performed by stratifying virtual patient representing pediatric-adolescent patients aged 2 years old to less than 18 years old into weight groups of 10kg units, and exploring maintenance doses of 40, 60, 80, 100, and 120 mg SC Q2W (from 10-week) in a virtual pediatric-adolescent patient population and comparing with exposure values achieved according to the 120 mg SC Q2W (from 10-week) maintenance dose regimen in an adult patient group.

Since a simulated partial Mayo and modified Mayo score did not show a difference compared to an administration dose and body weight, an infliximab administration dose used in the Example 2-1 clinical trial was selected based on a PK simulation result.

The PK-PD model constructed based on the above data can be used to simulate a subcutaneous administration result for a pediatric patient with an indication for infliximab (e.g., rheumatoid arthritis, ulcerative colitis, Crohn's disease, plaque psoriasis, psoriatic arthritis, or ankylosing spondylitis).

In a population PK and PK-PD modeling analysis for a UC patient, a Mayo score was included in an analysis target as well as a safety analysis in each indication (CD, RA, and AS). A final PK model was performed as a two-compartment model with linear elimination from a central compartment where infliximab infusion occurs and a depot compartment having a primary absorption rate to the central compartment. A covariate relationship between disease duration and a baseline Mayo score was applied to the model.

### Example 2-3. Simulation Result for Determination of Doses for Pediatric-Adolescent UC Patients

A simulation result of a PK parameter according to weight strata was within a PK equivalence margin of 80% to 125% at doses of 120 mg, 60 mg, and 40 mg (see Tables 1 to 3).

Furthermore, a simulated PK profile for a low-dose and high-dose group is shown in Fig. 1. Among them, since higher drug exposure was observed in a group in which a pediatric-adolescent body weight is 10-20 kg (60 mg infliximab Q2W maintenance dose) and a group in which a pediatric-adolescent body weight is 40-60 kg (120 mg infliximab Q2W maintenance dose) than in an adult group (120 mg Q2W maintenance dose), an additional PK simulation was conducted.

When compared to a drug exposure observed in adult patients receiving a 240 mg infliximab Q2W maintenance dose in NCT02883452, NCT04205643, and NCT03945019 clinical trials, it was shown that subcutaneously administering infliximab at 60 mg or 120 mg Q2W to a pediatric adolescent showed lower drug exposure in AUC and Cmax. Since no safety issue was observed when administering a 240 mg Q2W maintenance dose in the adult clinical trial result, the administration dose proposed in the Example 2-1 is predicted to be appropriate in terms of safety.

Based on a simulation result, a subcutaneous administration method for a pediatric-adolescent UC patient exposed to a minimum drug while showing a maximum efficacy is as follows:
* High-dose group
   - Administration of infliximab 120 mg via the SC route every 2 weeks from 10-week to 54-week (body weight of a patient ≥ 40 kg)
   - Administration of infliximab 60 mg via the SC route every 2 weeks from 10-week to 54-week (body weight of a patient < 40 kg)
* Low-dose group
   - Administration of infliximab 60 mg via the SC route every 2 weeks from 10-week to 54-week (body weight of a patient ≥ 40 kg)
   - Administration of infliximab 40 mg via the SC route every 2 weeks from 10-week to 54-week (body weight of a patient < 40 kg)

### Example 3. Stability of Composition

In order to confirm whether a composition subcutaneously administered in the Examples 1 and 2 has stability even when the composition contains a high concentration of infliximab, a formulation having a composition of Table 4 below was prepared, and stability thereof was measured after being stored for 0 weeks, 4 weeks, 8 weeks, and 12 weeks at a temperature of 5°C and 25°C.

**[Table 4]**

| **Composition** | | | |
|---|---|---|---|
| **Antibody** | **Surfactant** | **Sugar alcohol** | **Buffer** |
| **220 mg/ml infliximab** | **0.05% w/v polvsorbate 80** | **3.8% w/v sorbitol** | **90 mM acetate** |

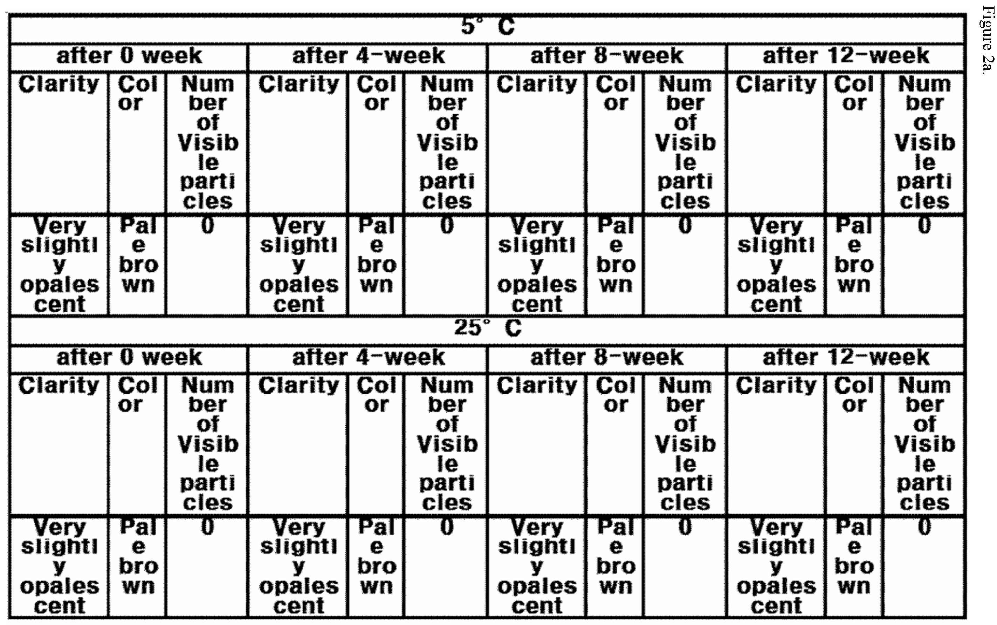

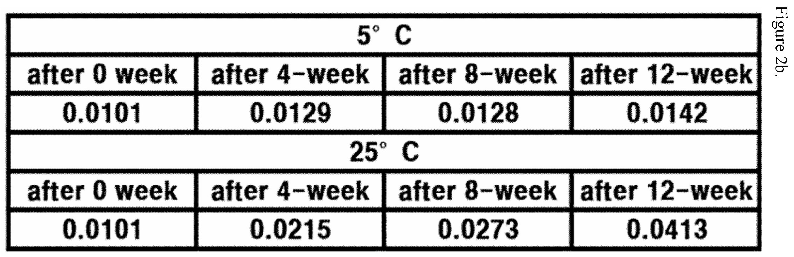

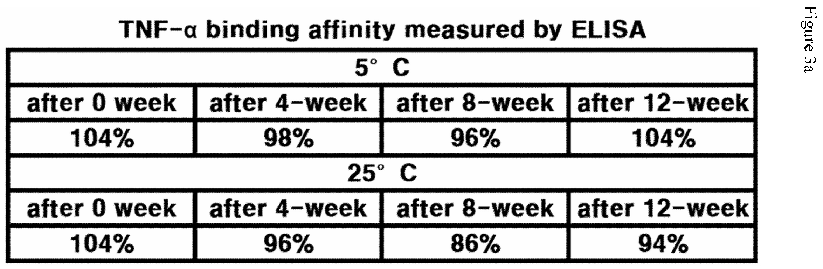

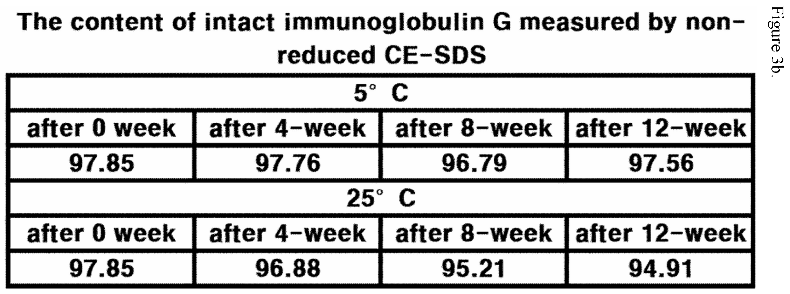

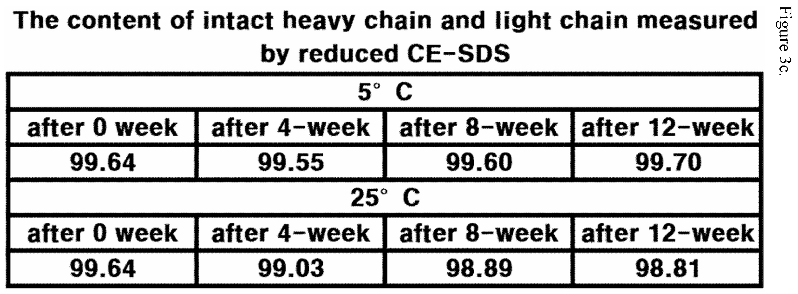

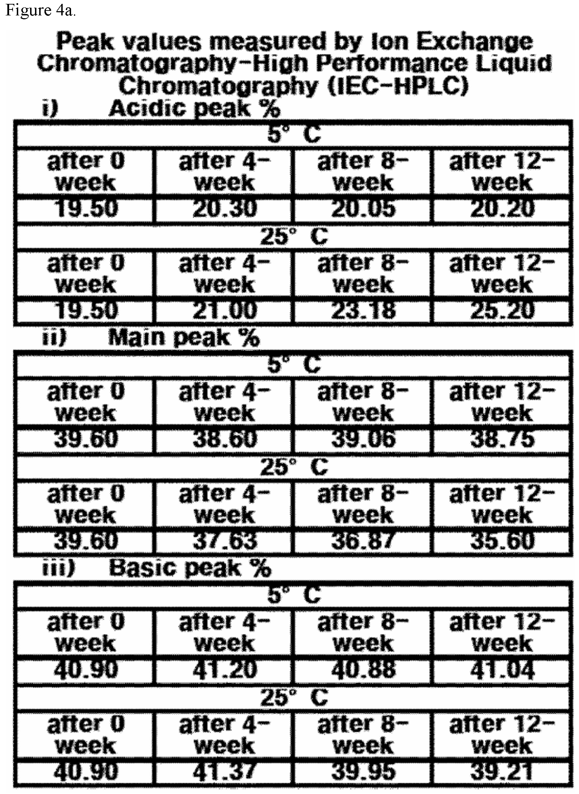

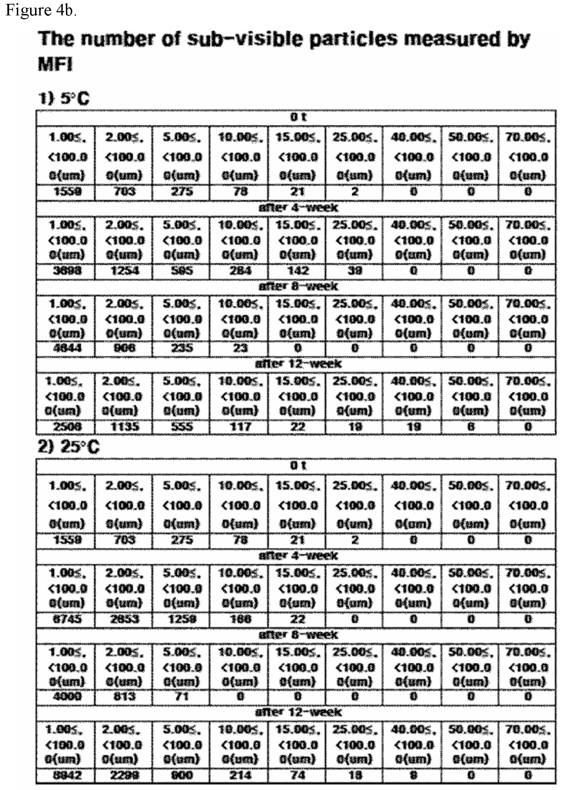

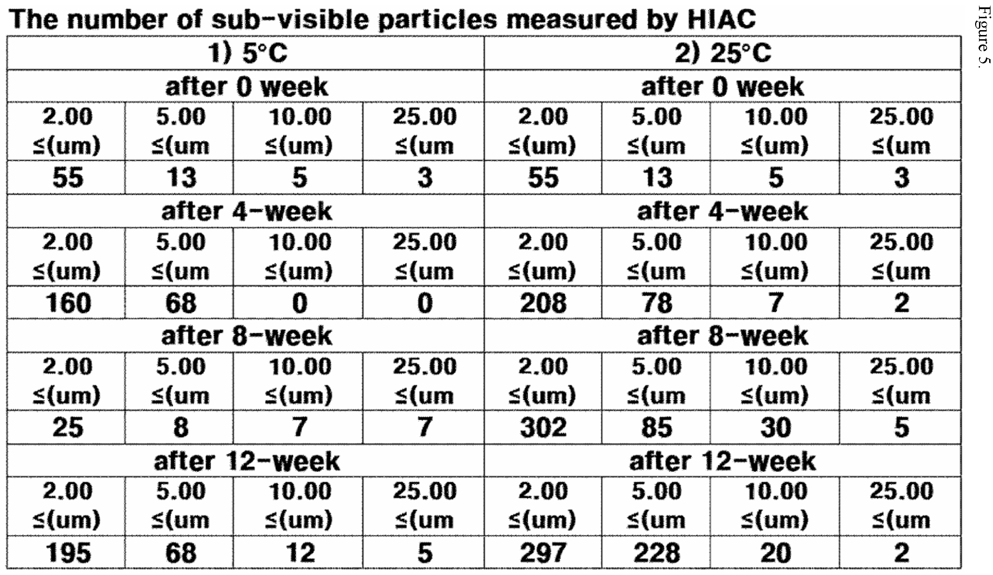

Since there was little change in color and turbidity even after 12 weeks passed, and almost no change in color and turbidity occurred even when the formulation contained a high-concentration antibody, the formulation having a composition of Table 4 was confirmed to have excellent stability (Fig. 2). Furthermore, a change was not significant when measuring the antigen-binding ability of infliximab by ELISA, and the range of change after 12 months was not significant even when measuring the immunoglobulin G content, the heavy chain, and the light chain content by CE-SDS. This demonstrates that the stability of the formulation having a composition of Table 4 is excellent (Fig. 3). Furthermore, the result measurement values of peaks and the number of sub-visible particles using an MFI analyzer or a HIAC particle counter after 12 months also confirmed the high stability of the formulation having a composition of Table 4 (see Figs. 4 and 5).

## Claims

1. A method for treating a TNFα-related disease, the method comprising administering, to a subject, a pharmaceutical composition comprising an anti-TNFα antibody or an antigen-binding fragment thereof,
wherein the anti-TNFα antibody or antigen-binding fragment thereof is subcutaneously administered to the subject at an actual dose of 40 to 300 mg at intervals of 1 to 8 weeks.

2. The method of claim 1, wherein the TNFα-related disease is selected from the group consisting of rheumatoid arthritis, ulcerative colitis, Crohn's disease, plaque psoriasis, psoriatic arthritis, and ankylosing spondylitis.

3. The method of claim 1 or 2, wherein the subject is a person who is 20 years old or less, 19 years old or less, 18 years old or less, 17 years old or less, 2 to 18 years old, 2 to 17 years old, or 6 to 17 years old.

4. The method of any one of claims 1 to 3, wherein the anti-TNFα antibody or antigen-binding fragment thereof is administered to the subject at a dose of 40 to 120 mg.

5. The method of any one of claims 1 to 4, wherein, when a condition of the subject is not improved or a therapeutic response is lost, a dose of the anti-TNFα antibody or antigen-binding fragment thereof is increased and administered.

6. The method of any one of claims 1 to 5, wherein the anti-TNFα antibody or antigen-binding fragment thereof is administered to the subject at a dose of 40 mg, 60 mg, or 120 mg.

7. The method of any one of claims 1 to 6, wherein the anti-TNFα antibody or antigen-binding fragment thereof is administered to the subject at intervals of 1, 2, 3, 4, 5, 6, 7, or 8 weeks.

8. The method of any one of claims 1 to 7, wherein the anti-TNFα antibody or antigen-binding fragment thereof is administered to the subject at intervals of 2 weeks.

9. The method of any one of claims 1 to 8, wherein, when a body weight of the subject is 40 kg or more, 60 to 120 mg of the anti-TNFα antibody or antigen-binding fragment thereof is administered, and
when a body weight of the subject is 40 kg or less, 40 to 60 mg of the anti-TNFα antibody or antigen-binding fragment thereof is administered.

10. The method of any one of claims 1 to 9, wherein, when a body weight of the subject is 40 kg or more, 120 mg of the anti-TNFα antibody or the antigen-binding fragment thereof is subcutaneously administered 5 times, and then, 60 mg of the anti-TNFα antibody or antigen-binding fragment thereof is administered from a 6-th administration.

11. The method of any one of claims 1 to 9, wherein, when a body weight of the subject is 40 kg or less, 60 mg of the anti-TNFα antibody or antigen-binding fragment thereof is subcutaneously administered 5 times, and then, 40 mg of the anti-TNFα antibody or the antigen-binding fragment thereof is administered from a 6-th administration.

12. The method of any one of claims 1 to 11, wherein the anti-TNFα antibody or antigen-binding fragment thereof is administered in combination with at least one selected from the group consisting of a disease-modifying antirheumatic drug (DMARD), a steroid, and an immunosuppressant.

13. The method of claim 12, wherein the disease-modifying antirheumatic drug (DMARD) is selected from the group consisting of methotrexate, leflunomide, sulfasalazine, and hydroxychloroquine,
the steroid is selected from the group consisting of corticosteroid, glucocorticoid, cortisol, mineralocorticoid, and aldosterone, and
the immunosuppressant is selected from the group consisting of azathioprine, 6-mercaptopurine, cyclosporin A, tacrolimus, mycophenolic acid, bredinin, an mTOR inhibitor, and an anti-lymphocyte antibody.

14. The method of any one of claims 1 to 13, wherein the subject is a subject who has been intravenously administered with the anti-TNFα antibody or antigen-binding fragment thereof at least once prior to subcutaneous administration.

15. The method of claim 14, wherein the subject is a subject who has been intravenously administered with the anti-TNFα antibody or antigen-binding fragment thereof two or three times prior to subcutaneous administration.

16. The method of claim 14, wherein:
a) in the case of a subject having a rheumatoid arthritis, the subject is a subject who has been intravenously administered with the anti-TNFα antibody or antigen-binding fragment thereof two times prior to subcutaneous administration; and
b) in the case of a subject having any one or more diseases selected from the group consisting of ulcerative colitis, Crohn's disease, plaque psoriasis, psoriatic arthritis, and ankylosing spondylitis, the subject is a subject who has been intravenously administered with the anti-TNFα antibody or antigen-binding fragment thereof two or three times prior to subcutaneous administration.

17. The method of claim 14, wherein the subject is a subject who, prior to subcutaneous administration, has been intravenously administered with the anti-TNFα antibody or antigen-binding fragment thereof two times at 0-week and 2-week, or
a subject who has received intravenous administration three times at 0-week, 2-week, and 6-week .

18. The method of claim 14, wherein the subject is a subject who has been intravenously administered with the anti-TNFα antibody or antigen-binding fragment thereof at a dose of 1 to 10 mg/kg per administration.

19. The method of claim 18, wherein the subject is a subject who has been intravenously administered with the anti-TNFα antibody or antigen-binding fragment thereof at a dose of 3 to 5 mg/kg per administration.

20. The method of claim 14, wherein:
a) in the case of a subject having a rheumatoid arthritis, the subject is a subject who has been intravenously administered with the anti-TNFα antibody or antigen-binding fragment thereof at a dose of 3 mg/kg per administration; and
b) in the case of a subject having any one or more diseases selected from the group consisting of ulcerative colitis, Crohn's disease, plaque psoriasis, psoriatic arthritis, and ankylosing spondylitis, the subject is a subject who has been intravenously administered with the anti-TNFα antibody or antigen-binding fragment thereof at a dose of 5 mg/kg per administration.

21. The method of claim 14, wherein a first subcutaneous administration is performed at 2 to 8 weeks after the last intravenous administration.

22. The method of claim 21, wherein a first subcutaneous administration is performed at 4 weeks after the last intravenous administration.

23. The method of any one of claims 1 to 22, wherein a trough blood concentration (C_{trough}) of the anti-TNFα antibody or antigen-binding fragment thereof is maintained at 0.01 µg/ml or more after subcutaneous administration to the subject.

24. The method of claim 23, wherein:
a) in the case of a subject having a rheumatoid arthritis, a trough blood concentration (C_{trough}) of the anti-TNFα antibody or antigen-binding fragment thereof is maintained at 1 µg/ml or more after subcutaneous administration; and
b) in the case of a subject having any one or more diseases selected from the group consisting of ulcerative colitis, Crohn's disease, plaque psoriasis, psoriatic arthritis, and ankylosing spondylitis, a trough blood concentration (C_{trough}) of the anti-TNFα antibody or antigen-binding fragment thereof is maintained at 5 µg/ml or more after subcutaneous administration.

25. The method of any one of claims 1 to 24, wherein, after subcutaneous administration, the subject has any one or more of the following characteristics:
a) a disease activity score in 28 joints (DAS28) being reduced by at least 2.0; or
b) a Crohn's disease activity index (CDAI) being reduced by at least 70.

26. The method of any one of claims 1 to 25, wherein the subject prior to subcutaneous administration has any one or more of the following characteristics:
a) a subject who has an insufficient response to a disease-modifying antirheumatic drug (DMARD) including methotrexate;
b) a subject who has not previously been treated with methotrexate or another DMARD;
c) a subject who exhibits an increase in a serological indicator related to severe axial symptoms and inflammation, which does not exhibit an adequate response to conventional treatment; or
d) a subject who does not exhibit an adequate response to a systemic therapy including methotrexate, cyclosporine, or psoralen ultraviolet A therapy (PUVA), or does not have tolerance to such a therapy, or for whom such a therapy is contraindicated.

27. The method of any one of claims 1 to 26, wherein the subject prior to subcutaneous administration has any one or more of the following characteristics:
a) a subject who does not exhibit an adequate response to treatment of a corticosteroid, 6-mercaptopurine, azathioprine, or an immunosuppressant or does not have tolerance to such a treatment, or for whom such treatment method is contraindicated; or
b) a subject who does not exhibit a response to conventional treatment including antibiotics, excretion, or immunosuppressive treatment.

28. The method of any one of claims 1 to 27, wherein the anti-TNFα antibody or antigen-binding fragment thereof comprises:
a light chain variable region comprising a CDR1 domain comprising an amino acid sequence of SEQ ID NO: 1, a CDR2 domain comprising an amino acid sequence represented by YAS, and a CDR3 domain comprising an amino acid sequence of SEQ ID NO: 2; and
a heavy chain variable region comprising a CDR1 domain comprising an amino acid sequence of SEQ ID NO: 3, a CDR2 domain comprising an amino acid sequence of SEQ ID NO: 4, and a CDR3 domain comprising an amino acid sequence of SEQ ID NO: 5.

29. The method of any one of claims 1 to 28, wherein the anti-TNFα antibody is infliximab.

30. The method of any one of claims 1 to 29, wherein the pharmaceutical composition comprising the anti-TNFα antibody or antigen-binding fragment thereof comprises:
(A) 90 to 220 mg/ml or 90 to 180 mg/ml of the anti-TNFα antibody or antigen-binding fragment thereof;
(B) 0.02 to 0.1 % (w/v) of polysorbate;
(C) 1 to 10 % (w/v) of sorbitol; and
(D) 1 to 90 mM or 1 to 50 mM of a buffer containing acetate.

31. The method of any one of claims 1 to 30, wherein the pharmaceutical composition comprising the anti-TNFα antibody or antigen-binding fragment thereof is filled in a sterile container, a pre-filled syringe, or an auto-injector.

32. A pharmaceutical composition for treating a TNFα-related disease, the pharmaceutical composition comprising an anti-TNFα antibody or an antigen-binding fragment thereof, wherein the anti-TNFα antibody or antigen-binding fragment thereof is subcutaneously administered at a dose of 40 to 300 mg at intervals of 1 to 8 weeks.

33. A kit comprising:
(a) a pharmaceutical composition comprising an anti-TNFα antibody or an antigen-binding fragment thereof; and
(b) an instruction indicating that the anti-TNFα antibody or antigen-binding fragment thereof is subcutaneously administered at a dose of 40 to 300 mg at intervals of 1 to 8 weeks to treat a subject with a TNFα-related disease.

34. A use of an anti-TNFα antibody or an antigen-binding fragment thereof for manufacturing a medicament for treating a TNFα-related disease by being administered to a subject, wherein the anti-TNFα antibody or antigen-binding fragment thereof is subcutaneously administered at a dose of 40 to 300 mg at intervals of 1 to 8 weeks.
